# EUROPEAN PATENT SPECIFICATION

(11) **EP 3 083 993 B1**
(45) Date of publication and mention of the grant of the patent: **19.06.2019**
(21) Application number: 14821622.9
(22) Date of filing: 19.12.2014
(51) Int. Cl.: C12Q 1/68

(54) **GENE METHYLATION BASED COLORECTAL CANCER DIAGNOSIS**
AUF GENMETHYLIERUNG BASIERENDE DIAGNOSE VON KOLOREKTALKARZINOM
DIAGNOSTIC DE CANCER COLORECTAL BASÉ SUR LA MÉTHYLATION GÉNIQUE

(30) Priority: 20.12.2013 EP 13198755
(43) Date of publication of application: 26.10.2016
(73) Proprietor: AIT Austrian Institute of Technology GmbH, 1210 Wien (AT)
(72) Inventor: WEINHÄUSEL, Andreas, A-7311 Neckenmarkt (AT); PULVERER, Walter, A-3386 Hafnerbach (AT)
(74) Representative: Sonn & Partner Patentanwälte
(86) International application number: PCT/EP2014/078792
(87) International publication number: WO 2015/091979

(56) References cited:
- WO-A1-2010/086389
- WO-A2-03/072821
- WO-A2-2012/047899
- S. C. GLOCKNER ET AL: "Methylation of TFPI2 in Stool DNA: A Potential Novel Biomarker for the Detection of Colorectal Cancer", CANCER RESEARCH, vol. 69, no. 11, 12 May 2009 (2009-05-12), pages 4691-4699, XP055103002, ISSN: 0008-5472, DOI: 10.1158/0008-5472.CAN-08-0142 cited in the application
- MEGAN M GARRITY-PARK ET AL: "Methylation Status of Genes in Non-Neoplastic Mucosa From Patients With Ulcerative Colitis-Associated Colorectal Cancer", THE AMERICAN JOURNAL OF GASTROENTEROLOGY, vol. 105, no. 7, 1 July 2010 (2010-07-01), pages 1610-1619, XP055103469, ISSN: 0002-9270, DOI: 10.1038/ajg.2010.22

## Description

The present invention relates to colorectal cancer diagnostic methods and means therefor.

Epigenetic instability of tumor-related genes (including oncogenes and tumor suppressor genes) is one of the molecular key events in the pathogenesis of cancer. DNA methylation which is one major cause of epigenetic instability is virtually present in all tumor types and has been proposed as a candidate for cancer biomarker. Premalignant lesions already show changes in their DNA methylation patterns which could serve as indicator for future disease breakout.

Colorectal cancer (CRC) is the third most common cause of cancer related deaths. The progression of CRC starts with the loss of the Adenomatous polyposis coli (*APC*) gene and followed by acquired mutations in *KRAS*, LOH of 17p and 18q as well as mutations in *TP53.* In many tumors global loss of DNA methylation and focal hypermethylation of CpG islands have been observed, causing genome instability and silencing of tumor suppressor genes, respectively. Standard treatment for T2 and T3 distal rectal cancer is preoperative short-term irradiation with the intention to enable sphincter-preserving surgery and reduce local recurrence rate.

CRC-specific mortality rates can substantially be reduced by early identification and treatment of the disease, preferably in a pre-malignant state. Colonoscopy is currently the primary screening tool for CRC, however also that methodology is limited in its effectiveness. Hence, that problem reveals the need for early identification and treatment of especially pre-cancerous colorectal lesions.

WO 2012/047899 A2 and WO 03/072821 A2 describe DNA hypermethylation in colorectal cancer.

Garrity-Park et al., The American Journal of Gastroenterology, 105(7)(2010): 1610 -01619, relates to an investigation on methylation status of genes of the non-neoplastic mucosa.

A number of differentially methylated genes as biomarkers have been discovered over years rather by chance than by rationality, e.g. methylation of TFPI2 in stool DNA (Glöckner et al., Cancer Research 2009; 69: 4691-4699). Albeit some of these methylation changes have the potential being useful markers for differentiation of specifically defined diagnostic questions, these would lack the power for successful delineation of various diagnostic constellations.

The goal of the present invention is to provide a colorectal cancer diagnostic assay and means, which improve on prior gene methylation testing.

The goal is achieved by the subject matter of the claims and the additional embodiments described herein. As known from the prior art, often a single marker does not provide sufficient reliability for diagnostic uses. Marker combinations have the potential to alleviate this problem. The present invention provides such new combinations, which provide safer colon cancer diagnostics. In the course of identifying new marker combinations, also new markers have been identified, which can alone or in combination with other diagnostic methods (not limited to biomarker methylation testing) be used to provide an indication towards colorectal cancer likelihood.

In particular, the invention provides a method of identifying a colorectal cancer in a sample comprising DNA from a subject, determining the methylation status of two or more genes comprising TFPI2 and PTGS2 and comparing the methylation status with a control sample, thereby identifying colorectal cancer DNA in the sample. Disclosed are methods for identifying methylation in one or more, e.g. 1, 2, 3, 4, 5, 6, 7, 8, 9, 10, 11, 12, 13, 14, 15, 16, 17, 18, 19, 20, 21, 22, 23, 24, 25, 26, 27 or 28, of the following genes selected from BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1, WT1 or combinations thereof in a sample of a subject to be tested for colorectal cancer. These markers have been developed and validated as DNA methylation signatures, allowing the detection of colorectal cancer (CRC) by analysis of the DNA methylation pattern.

In a further aspect, the present invention relates to a set of nucleic acid primers or hybridization probes being specific for a potentially methylated region of marker genes being suitable to diagnose or predict colorectal cancer, with the set comprising probes or primers for genes: a) BOLL, CDX1, CLIC4, DCC, ESR1, GATA4, GDNF, HLA-G, JUB, MY-OD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 and WT1; b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 and JUB; f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 and S100A2; h) TFPI2, DCC, PTGS2, TJP2, PITX2; i) TFPI2, DCC, PTGS2, TJP2; j) TFPI2, DCC, PTGS2; k) TFPI2, PTGS2; or probes or primers for at least 50%, preferably at least 60%, at least 70%, at least 80%, at least 90%, 100% of the genes of anyone of the above a), b), f), h), i), j) to t), with the proviso that at least the 2 genes selected from TFPI2 and PTGS2 are represented, and any of these sets comprises not more than 350 probes or primer pairs. Disclosed is a set of nucleic acids, such as primers and/or probes, suitable for performing the inventive method, which nucleic acids, primers and/or probes are specific for targeting a potentially methylated region in a DNA molecule of one or more of the genes selected from BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1, WT1. Such a set can be a set of PCR primers or a microarray comprising the probes.

Also disclosed is a set of nucleic acids, such as primers and/or probes, suitable for performing the inventive method, which nucleic acids, primers and/or probes are specific for targeting a potentially methylated region in a DNA molecule of one or more of the genes selected from GATA4, WT1, TFPI2, SEZ6L, TMEFF2, DCC, BOLL, PITX2, GDNF, TWIST1, S100A2, DAPK1, ZNF256, RARB, SFRP2, SPARC, CALCA. These markers provide particularly high diagnostic power in case of body fluid, e.g. serum, samples.

The following detailed description relates to all aspects of the invention likewise: The inventive method can be performed by any embodiment of set or the primers and/or probes and the inventive set can be used for or be suitable for, i.e. comprising the means for performing, any of the inventive methods. Of course all described embodiments can be combined with each other as is apparent to a skilled practitioner.

The terms "colon cancer" and "colorectal cancer" are used interchangeably herein.

As mentioned above, some methylation markers, such as TFPI2 in stool have been known previously. However, methods using a previously known marker could be improved by combination with one or more of the other 27 markers given above. In addition, the invention provides for the first time also the use of such a known marker, alone or in combination with other markers, in new sample types, including blood or serum from the circulation (i.e. not stool blood) of the patient. Other markers, that were known in special applications, and the use of which in detecting colorectal cancer could be improved by the present invention, e.g. as mentioned above or explained in more detail below, are TWIST1 (Okada et al., Genes, Chromosomes & Cancer 49:452-462 (2010)) WT1 (Hiltunen et al., British Journal of Cancer (1997) 76(9), 1124-1130) and ESR1 (Woodson et al., Cancer Epidemiol Biomarkers Prev. 2005;14:1219-1223).

It should be understood that any methylation specific region which is readily known to the skilled man in the art from prior publications or available databases (e.g. PubMeth in the www at www.pubmeth.org or ENCODE of the Encyclopedia of DNA Elements (ENCODE) Consortium of the NHGRI, using the UCSC Genome Browser in the www at genome.ucsc.edu), especially using human genome version hg19 of Feb. 2009, can be used according to the present invention.

One advantage making DNA methylation an attractive target for diagnosis, is the fact that cell free methylated DNA can be detected in body-fluids like blood, plasma, serum, sputum, and urine from patients with cancerous neoplastic conditions and disease. It may also be detected in non-invasive samples, including stool. Stool may comprise cellular material from intestinal villi and tumor tissue allowing the detection of the inventive methylation markers due to the presence of tumor DNA in this tissue material. Preferably the inventive method is performed on such colon tissue found in stool. It may or may not be performed on stool blood or on samples comprising stool blood. Especially methylation of marker TFPI2 is preferably not performed on stool blood. Also, it is also possible to perform the inventive method on tumour tissue obtained from the colorectal wall, e.g. biopsy tissue. Control samples can be of the same tissues or a different tissue, e.g. a different tissue selected from the same as above independent on the patient sample under investigation, especially body fluid or tissue samples.

Cancer specific DNA methylation pattern can be found in cell free DNA derived from serum, as tumors release substantial amounts of their DNA into the bloodstream. Especially tumors which are flushed thoroughly by blood release considerable amounts of tumor DNA into the blood stream. Thus DNA methylation is well suited for noninvasive detection in materials like serum, plasma (blood plasma) and blood.

It is possible to determine the colorectal cancerous condition from methylated DNA that is obtained from urine, blood, plasma, serum. Other sample types are e.g. tissue samples. The inventive method may comprise the step of isolating DNA from urine, blood, plasma or serum. The inventive set or kit may comprise means for isolation of DNA from said sources, such as a DNA isolation kit. The kit may also comprise means to isolate blood, plasma or serum from the circulatory system of the subject, such as a syringe, or a biopsy extractor.

The inventive methylation testing in the inventive marker genes may be in comparison to a normal reference sample. Any one of the above sample types may be used as reference sample, selected independently from the sample of the tested subject.

The subject may have had a colorectal cancer or he may have had no history of colorectal cancer, in particular if the subject is suspected of having colorectal cancer. The invention may be used as a routine test even without any suspect of the subject having colorectal cancer. In particular preferred the invention is not used for follow up investigations in order to determine efficacy of an anti-colorectal cancer treatment or it may be used for such a follow up investigation.

The present invention can also be used for prognosis of cancer, in particular a prediction of the progression of a colorectal cancer. A particularly preferred use of the invention is to perform a diagnosis or prognosis of a cancer, which may be a metastasising colorectal cancer.

In the context of the present invention "prognosis", "prediction" or "predicting" should not be understood in an absolute sense, as in a certainty that an individual will develop cancer (including cancer progression), but as an increased risk or likelihood to develop cancer or of cancer progression. "Prognosis" is also used in the context of the invention for predicting cancer progression, in particular to predict therapeutic results of a certain therapy of the colorectal cancer. The prognosis of a therapy can e.g. be used to predict a chance of success (e.g. treating cancer with cancerous DNA methylation marker states being below detection levels) or chance of reducing the severity of the disease to a certain level. The inventive marker sets may also be used to monitor a patient for the emergence of therapeutic results or positive disease progressions.

Likewise, "diagnosing" may or may not be used to provide information of a 100% sure cancer presence, but is usually used to describe a risk or likelihood of cancer presence. Such a risk or likelihood may be at least 60%, at least 70%, at least 80% at least 90% at least 95% or at least 98% probability.

Furthermore the presented data can be used as starting point to evaluate the methylation value of DNA derived from serum or other minimal invasive methods for the early diagnosis of CRC.

If only limiting amounts of DNA were available for analyses an amplification protocol can be used enabling selective amplification of the methylated DNA fraction prior methylation testing.

The inventive diagnostic marker genes have been mentioned above and are listed in any one of tables 2, 3 and 4. Additional markers that can be used are provided in table 1. Such additional markers, e.g. those of table 1 but not being limited to table 1, may comprise positive or negative controls for differential DNA methylation. These controls can be used to baseline or normalize the methylation measurement method. Further markers that may be used as auxiliary diagnostic marker genes or control are described in WO 2010/086389 A1.

The inventive marker genes to be used in methylation testing for colorectal cancer diagnosis are defined in claim 1. Further marker genes may be BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1, WT1 and especially combinations of these markers.

Especially preferred combinations are of any 2, 3, 4, 5, 6, 7, 8, 9, 10 or more genes selected from ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3, JUB. Experimental data on differential methylation is shown in table 2. The marker genes according to table 2 are ranked in order of preference. Especially preferred are the first 2, 3, 4, 5, 6, 7, 8, 9, or 10 makers of table 2.

Especially preferred combinations are of any 2, 3, 4, 5, 6, 7, 8, 9, 10 or more genes selected from TMEFF2, PITX2, TWIST1, ESR1, BOLL, TFPI2, WT1, GDNF, HLA-G, PENK, SEZ6L, SFRP2, TCEB2, RARB. Experimental data on differential methylation is shown in table 3. The marker genes according to table 3 are ranked in order of preference. Especially preferred are the first 2, 3, 4, 5, 6, 7, 8, 9, or 10 makers of table 3.

Especially preferred combinations are of any 2, 3, 4, 5, 6, 7, 8, 9, 10 or more genes selected from TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MY-OD1, S100A2. Experimental data on differential methylation is shown in table 4. The marker genes according to table 4 are ranked in order of preference. Especially preferred are the first 2, 3, 4, 5, 6, 7, 8, 9, or 10 makers of table 4.

Especially preferred combinations are of any 2, 3, 4, 5, 6, 7, 8, 9, 10 or more genes selected from GATA4, WT1, TFPI2, SEZ6L, TMEFF2, DCC, BOLL, PITX2, GDNF, TWIST1, S100A2, DAPK1, ZNF256, RARB, SFRP2, SPARC, CALCA , especially preferred selected from GATA4, WT1, TFPI2, SEZ6L, TMEFF2, DCC, BOLL, PITX2, GDNF. Experimental data on differential methylation is shown in tables 11-17. The marker genes according to table 11 are ranked in order of preference. Especially preferred are the first 2, 3, 4, 5, 6, 7, 8, 9, or 10 makers of table 11. Preferably such a combination comprises TMEFF2 and/or TWIST1, especially TMEFF2. Preferably these markers are used for body fluid, e.g. serum, analysis.

Disclosed is the use of the methylation dependent markers selected from the genes of one or more of following groups:
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 and WT1;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 and JUB;
c) TFPI2 and TJP2;
d) TMEFF2, PITX2, TWIST1, ESR1, BOLL, TFPI2, WT1, GDNF, HLA-G, PENK, SEZ6L, SFRP2, TCEB2 and RARB;
e) TMEFF2, TWIST1, PITX2 and TFPI2;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 and S100A2;
g) TFPI2, TMEFF2, TWIST1, ESR1, PITX2, DCC, WT1, SEZ6L, BOLL and SFRP2;
h) TFPI2, DCC, PTGS2, TJP2, PITX2;
i) TFPI2, DCC, PTGS2, TJP2;
j) TFPI2, DCC, PTGS2;
k) TFPI2, PTGS2;
l) TMEFF2, PITX2, TWIST1, SFRP2, RARB, ZNF256, SPARC, DAPK1, CALCA, S100A2;
m) TMEFF2, PITX2, TWIST1, SFRP2, ZNF256, SPARC, DAPK1;
n) TMEFF2, PITX2, TWIST1, SPARC, S100A2;
o) TMEFF2, TWIST1;
p) GATA4, WT1, TFPI2, SEZ6L, TMEFF2, DCC, BOLL, PITX2, GDNF;
q) TMEFF2, PITX2, TWIST1;
r) TMEFF2, TWIST1, PTX2, S100A2;
s) DAPK1, TMEFF2, PITX2, S100A2, ZNF256, RARB, SFRP2;
t) DAPK1, TMEFF2, PITX2, S100A2, SPARC, CALCA, TWIST1, ZNF256, RARB, SFRP2;
or a group of markers comprising at least 50%, preferably at least 60%, at least 70%, at least 80%, at least 90%, 100% of the genes of anyone of the above groups a) to t), preferably wherein, for a given group, at least 2 genes are used.

These groups, including the groups of tables 2, 3, 4, 11-17 include marker combinations with a high probability of a correct colorectal cancer diagnosis. A skilled reader understand, that it is possible to use only a fraction of the markers of any of these groups and lists and tables, which may have a reduced but still sufficient probability of a correct colorectal cancer diagnosis. The present invention does not exclude such less efficient diagnostic methods. In addition, the reduced efficiency of a reduced marker combination may be compensated by any other marker known in the art, such as disclosed in WO 2010/086389 A1. Such use of auxiliary markers may be validated on samples of a known colorectal cancer positive and/or negative samples - also as disclosed in WO 2010/086389 A1.

Such a validation may comprise the colorectal cancer positive and/or negative samples, preferably at least 4, at least 6, at least 8 colorectal cancer positive and/or negative samples and determining the methylation status in the genes to be validated. Statistical methods, including a correlation, may be used for this validation.

The inventive selection of auxiliary marker genes can be made by any (known) classification method to obtain a set of markers with the given diagnostic (or also prognostic) value to categorize colorectal cancer. Such methods include class comparisons wherein a specific p-value is selected, e.g. a p-value below 0.1, preferably below 0.08, more preferred below 0.06, in particular preferred below 0.05, below 0.04, below 0.02, most preferred below 0.01.

Preferably the correlated results for each gene are rated by their correct correlation to colorectal cancer positive state, preferably by p-value test or t-value test or F-test. Rated (best first, i.e. low p- or t-value) markers are the subsequently selected and added to the marker combination until a certain diagnostic value is reached, e.g. the herein mentioned at least 60%, at least 70%, at least 80%, at least 90% or at least 95% (or more) correct classification of colorectal cancer.

Class Comparison procedures include identification of genes that were differentially methylated among the two classes using a random-variance t-test. The random-variance t-test is an improvement over the standard separate t-test as it permits sharing information among genes about within-class variation without assuming that all genes have the same variance (Wright G.W. and Simon R, Bioinformatics 19:2448-2455,2003). Genes were considered statistically significant if their p value was less than a certain value, e.g. 0.1 or 0.01. A stringent significance threshold can be used to limit the number of false positive findings. A global test can also be performed to determine whether the methylation profiles differed between the classes by permuting the labels of which arrays corresponded to which classes. For each permutation, the p-values can be re-computed and the number of genes significant at the e.g. 0.01 level can be noted. The proportion of the permutations that give at least as many significant genes as with the actual data is then the significance level of the global test. If there are more than 2 classes, then the "F-test" instead of the "t-test" should be used.

Class Prediction includes the step of specifying a significance level to be used for determining the genes that will be included in the subset. Genes that are differentially methylated between the classes at a univariate parametric significance level less than the specified threshold are included in the set. It doesn't matter whether the specified significance level is small enough to exclude enough false discoveries. In some problems better prediction can be achieved by being more liberal about the gene sets used as features. The sets may be more biologically interpretable and clinically applicable, however, if fewer genes are included.

The gene combination or set can also be optimized for a specific sample type in which the methylated DNA is tested. Such samples include blood, plasma, serum, urine, saliva, hair, skin, stool, tissues, in particular tissues of the colon, especially tissues being suspected of having colon cancer cells, such as colon polyps. The sample may be obtained from a patient to be diagnosed.

To prevent increase of the number of the members of the subset, only marker genes with at least a significance value of at most 0.1, preferably at most 0.8, even more preferred at most 0.6, at most 0.5, at most 0.4, at most 0.2, or more preferred at most 0.01 are selected.

Since the combination should be small, it is preferred that not more than 350, not more than 300, not more than 250, not more than 200, not more than 150, not more than 100, not more than 80, not more than 60, or not more than 40, preferably not more than 30, in particular preferred not more than 20, marker genes are used according to the inventive method or in the inventive set, not counting controls for methylation testing as mentioned above. In particular the set of the present invention provides less primer pairs /and or probes than these numbers in order to reduce manufacturing costs in addition to the above reasons.

Especially preferred combination of the inventive genes for methylation testing in the diagnosis are TMEFF2, ESR1 and TWIST1; Especially preferred the one or more genes comprise a combination of TMEFF2 and ESR1; a combination of TMEFF2 and TWIST1; or a combination of ESR1 and TWIST1. Further preferred combinations comprise any 1, 2, 3, 4, 5, 6, 7, 8, 9 or 10 or more markers of tables 2, 3, 4 and 17 having a p-value <10⁻⁷.

Especially preferred, the one or more genes comprise TFPI2 and/or TJP2. These markers are particularly useful as they can show a high fold change and allow an efficient detection of differential methylation.

Further preferred, the one or more genes comprise 1, 2, 3, 4, 5, 6, 7, 8, 9, 10 or more of BOLL, GDNF, HLA-G, PENK, PITX2, RARB, SEZ6L, SFRP2, TMEFF2.

Also preferred, the one or more genes comprise one or more of ESR1, TFPI2, WT1, TWIST1, PTGS2. These markers are especially preferred when used to analyse samples from the circulatory system of a subject and/or when used to detect the methylation status at the special methylation region of the chromosomal locus given in table 1 or as defined by the amplification product of the primer pairs given for these markers in table 1, or a regions at most 1000 nucleotides in length apart from these regions. Any one of these, but especially PTGS2, was capable to improve the correct classification of any other marker given in the tables herein. The gene markers may be used in a combination with any additional 1, 2, 3, 4, 5, or more gene markers provided herein.

Of course any one of the preferred genes or gene combinations can be further combined with any one of the above mentioned or claimed other single genes, groups, lists or tables 2, 3, 4 and/or any one of tables 11-17.

The methylation status can be determined by any method known in the art including methylation dependent bisulfite deamination (and consequently the identification of mC - methylated C - changes by any known methods, including PCR and hybridization techniques). Preferably, the methylation status is determined by methylation specific PCR analysis, methylation specific digestion analysis and either or both of hybridisation analysis to non-digested or digested fragments or PCR amplification analysis of non-digested fragments. The methylation status can also be determined by any probes suitable for determining the methylation status including DNA, RNA, PNA, LNA probes which optionally may further include methylation specific moieties.

As further explained below the methylation status can be particularly determined by using hybridisation probes or amplification primer (preferably PCR primers) specific for methylated regions of the inventive marker genes. Discrimination between methylated and non-methylated genes, including the determination of the methylation amount or ratio, can be performed by using e.g. either one of these tools.

The determination using only specific primers aims at specifically amplifying methylated (or in the alternative non-methylated) DNA. This can be facilitated by using (methylation dependent) bisulfite deamination, methylation specific enzymes or by using methylation specific nucleases to digest methylated (or alternatively non-methylated) regions - and consequently only the non-methylated (or alternatively methylated) DNA is obtained. By using a genome chip (or simply a gene chip including hybridization probes for all genes of interest such as all 359 marker genes), all amplification or non-digested products are detected. I.e. discrimination between methylated and non-methylated states as well as gene selection (the inventive set or subset) is before the step of detection on a chip. A preferred method of using the inventive nucleic acid for the inventive methylation testing of the marker genes is PCR, especially qPCR, using primer pairs for potentially methylated regions in these gene. Such testing can be performed for any marker as described in any example.

Alternatively it is possible to use universal primers and amplify a multitude of potentially methylated genetic regions (including the genetic markers of the invention) which are, as described either methylation specific amplified or digested, and then use a set of hybridisation probes for the characteristic markers on e.g. a chip for detection. E.g. gene selection is performed on the chip.

Either set, a set of probes or a set of primers, can be used to obtain the relevant methylation data of the genes of the present invention. Of course, both sets can be used.

The method according to the present invention may be performed by any method suitable for the detection of methylation of the marker genes. In order to provide a robust and optionally re-useable test format, the determination of the gene methylation is preferably performed with a DNA-chip, real-time PCR, or a combination thereof. The DNA chip can be a commercially available general gene chip (also comprising a number of spots for the detection of genes not related to the present method) or a chip specifically designed for the method according to the present invention (which predominantly comprises marker gene detection spots).

Preferably the methylated DNA of the sample is detected by a multiplexed hybridization reaction. In further embodiments a methylated DNA is preamplified prior to hybridization, preferably also prior to methylation specific amplification, or digestion. Preferably, also the amplification reaction is multiplexed (e.g. multiplex PCR).

Preferred DNA methylation analyses use bisulfite deamination-based methylation detection or methylation sensitive restriction enzymes. Preferably the restriction enzyme-based strategy is used for elucidation of DNA methylation changes. Further methods to determine methylated DNA are e.g. given in EP 1 369 493 A1 or US 6,605,432. Combining restriction digestion and multiplex PCR amplification with a targeted microarray-hybridization is a particular advantageous strategy to perform the inventive methylation test using the inventive markers. A microarray-hybridization step can be used for reading out the PCR results. For the analysis of the hybridization data statistical approaches for class comparisons and class prediction can be used.

The inventive methods (for the screening of subsets or for diagnosis or prognosis of a disease or tumor type) are particularly suitable to detect low amounts of methylated DNA of the inventive marker genes. Preferably the DNA amount in the sample is below 500ng, below 400ng, below 300ng, below 200ng, below 100ng, below 50ng or even below 25ng. The inventive method is particularly suitable to detect low concentrations of methylated DNA of the inventive marker genes. Preferably the DNA amount in the sample is below 500ng, below 400ng, below 300ng, below 200ng, below 100ng, below 50ng or even below 25ng, per ml sample.

The inventive method may comprise comparing the methylation status with the status of a confirmed colorectal cancer positive and/or negative state. The control may be of a healthy subject or devoid of significant cancer signatures, such as healthy tissue or PBMCs, of a healthy subject but also of the subject to be diagnosed.

Preferably, the sample of the subject is a colorectal tissue sample, preferably of a colorectal polyp, or a blood, plasma or serum sample, especially a blood, plasma or serum sample taken from the circulatory system of the subject. Alternatively it may also be a blood sample from the stool. The circulatory system is an organ system that permits blood and lymph circulation. It transports at least serum and contains DNA. In case of colorectal cancer, the cancer cells discharge DNA, including methylated or demethylated DNA of the inventive marker genes, which can be detected from these sources.

In particular preferred embodiments, the inventive method or set utilizes detection of differential methylation, as compared to a control. The control sample may be a colorectal tissue sample, a sample adjacent to a colorectal polyp or cancer tissue, or a blood or serum sample, especially a sample comprising blood cells, such as PBMCs. Such a control may be of a healthy control, a diseased state control. The healthy control may be of a healthy subject or of the subject to be diagnosed, if from a containing a known colon cancer positive or negative state, such as PBMCs.

In particular preferred a negative control is used. The inventive diagnosis may be based on increased methylation of the inventive marker genes. In comparison with other controls a decreased methylation may be detected. Marker S100A2 preferably has a decreased methylation.

In preferred embodiments of the invention, combinable with any one of the other embodiments and gene selections mentioned above, the methylation status of said genes is determined in an upstream region of the open reading frame of the marker genes, in particular a promoter region. In addition or alternatively, it may be determined in a) a genetic locus defined by the primer pairs of SEQ ID NO: 1 to 96 and/or b) the chromosomal locus as identified in table 1; or c) a locus within at most 1000 nucleotides in length distanced from said locus a) or b). The locus for detection may be within at most 800, at most 600, at most 500, at most 400, at most 300, at most 200, or at most 100, nucleotides in length distanced from said locus a) or b). Further loci are described by the PCR products in table 1 of WO2010/086389 A1, especially genetic regions defined by SEQ ID NOs 1081 to 1440 of WO2010/086389 A1, including the adjacent up to 500 base pairs, preferably up to 300, up to 200, up to 100, up to 50 or up to 10 adjacent, corresponding to gene marker IDs 1 to 359 of table 1 of WO2010/086389 A1, respectively.

Examples of specific probes or primers are given in table 1 with reference to the sequence listing, SEQ ID NOs 1 to 96, which form especially preferred embodiments of the invention.

In a further aspect, the present invention provides a set of nucleic acid primers or hybridization probes being specific for a potentially methylated region of marker genes being suitable to diagnose or predict colorectal cancer, with the set comprising probes or primers for genes: a) BOLL, CDX1, CLIC4, DCC, ESR1, GATA4, GDNF, HLA-G, JUB, MY-OD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 and WT1; b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 and JUB; f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 and S100A2; h) TFPI2, DCC, PTGS2, TJP2, PITX2; i) TFPI2, DCC, PTGS2, TJP2; j) TFPI2, DCC, PTGS2; k) TFPI2, PTGS2; or probes or primers for at least 50%, preferably at least 60%, at least 70%, at least 80%, at least 90%, 100% of the genes of anyone of the above a), b), f), h), i), j) to t), with the proviso that at least the 2 genes selected from TFPI2 and PTGS2 are represented, and any of these sets comprises not more than 350 probes or primer pairs. Disclosed is set of nucleic acid primers, primer pairs or hybridization probes being specific for a potentially methylated region of marker genes being suitable to diagnose or predict colorectal cancer according to any method of the invention, E.g. the set may comprise probes or primers or primer pairs for genes of the following lists:
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 and WT1;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 and JUB;
c) TFPI2 and TJP2;
d) TMEFF2, PITX2, TWIST1, ESR1, BOLL, TFPI2, WT1, GDNF, HLA-G, PENK, SEZ6L, SFRP2, TCEB2 and RARB;
e) TMEFF2, TWIST1, PITX2 and TFPI2;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 and S100A2;
g) TFPI2, TMEFF2, TWIST1, ESR1, PITX2, DCC, WT1, SEZ6L, BOLL and SFRP2;
h) TFPI2, DCC, PTGS2, TJP2, PITX2;
i) TFPI2, DCC, PTGS2, TJP2;
j) TFPI2, DCC, PTGS2;
k) TFPI2, PTGS2;
l) TMEFF2, PITX2, TWIST1, SFRP2, RARB, ZNF256, SPARC, DAPK1, CALCA, S100A2;
m) TMEFF2, PITX2, TWIST1, SFRP2, ZNF256, SPARC, DAPK1;
n) TMEFF2, PITX2, TWIST1, SPARC, S100A2;
o) TMEFF2, TWIST1;
p) GATA4, WT1, TFPI2, SEZ6L, TMEFF2, DCC, BOLL, PITX2, GDNF;
q) TMEFF2, PITX2, TWIST1;
r) TMEFF2, TWIST1, PTX2, S100A2;
s) DAPK1, TMEFF2, PITX2, S100A2, ZNF256, RARB, SFRP2;
t) DAPK1, TMEFF2, PITX2, S100A2, SPARC, CALCA, TWIST1, ZNF256, RARB, SFRP2;
or probes or primers for at least 50%, preferably at least 60%, at least 70%, at least 80%, at least 90%, 100% of the genes of anyone of the above a) to t), with the proviso that at least 2 genes are represented, or any other combination mentioned above. Preferably the set comprises not more than 350 probes or primer pairs.

In the inventive method or set in preferred embodiments, not more than 350 probes or primer pairs, especially not more than any number selected from 320, 300, 280, 260, 240, 220, 200, 180, 160, 140, 120, 100, 80, 70, 60, 50, 40, or 30 or any range therein are used.

Preferably, the primer pairs and probes are specific for a methylated upstream region of the open reading frame of the marker genes, in particular a promoter region; or specific for a) a genetic locus defined by the primer pairs of SEQ ID NO: 1 to 96 and/or b) the chromosomal locus as identified in table 1; or c) a locus within at most 1000 nucleotides in length distanced from said locus a) or b). Especially preferred, the probes or primers are of SEQ ID NOs 1 to 96.

Set according to the invention may be provided in a kit together with a methylation specific restriction enzyme and/or a reagent for bisulfite nucleotide deamination; and/or wherein the set comprises probes on a microarray.

Preferably the set is provided on a solid surface, in particular a chip, whereon the primers or probes can be immobilized. Solid surfaces or chips may be of any material suitable for the immobilization of biomolecules such as the moieties, including glass, modified glass (aldehyde modified) or metal chips.

The primers or probes can also be provided as such, including lyophilized forms or being in solution, preferably with suitable buffers. The probes and primers can of course be provided in a suitable container, e.g. a tube or micro tube.

The inventive marker set, including certain disclosed subsets, which can be identified with the methods disclosed herein, are suitable to distinguish between colorectal cancer, adjacent tissue and blood, in particular for diagnostic or prognostic uses.

The present invention is further illustrated by the following examples, without being limited to these embodiments of the invention.

### Example 1: Selection of patients, study design and treatment

For the methylation screening experiments DNA was isolated from 22 fresh frozen tumor samples. In addition the methylation analyses were conducted using 8 DNA samples isolated from blood of healthy donors (4 female, 4 male). For qPCR confirmation experiments DNA of 18 fresh frozen adenocarcinoma samples, 18 samples from the unaffected adjacent tissue and 12 PBMCs was used. Upon tumor resection and histopathological examination, representative tissue samples of tumors were immediately shock frozen in aliquots and stored in liquid nitrogen and used for discovery of DNA-methylation differences in cancerous vs. non-cancerous tissues.

DNA from PBMCs was provided by healthy volunteers and served as normal control samples for the experiments. Total DNA was isolated from fresh frozen tissue samples using commercially available kits (DNeasy; Qiagen, Hilden, Germany). Total DNA yield and purity was measured by UV spectroscopy (Nanodrop ND-1000 Spectrophotometer; Nanodrop Technologies, Wilmington, DE, USA) .

### Example 2: DNA methylation arrays

DNA (600ng) from fresh frozen tumorous tissue (n=22,) and DNA from peripheral blood (n=8, 4 male and 4 female) was digested using a mixture of methylation specific restriction enzymes (MSRE) containing HpaII (recognition site: CCGG), Hin6I (recognition site: GCGC; both Fermentas, St. Leon-Rot, Germany), AciI (recognition site: CCGC) and HpyCH4IV (recognition site: ACGT; both NEB, Frankfurt am Main, Germany). Digestion was conducted at 37 °C overnight, using 3U of each MSRE to cleave unmethylated DNA. Specific amplification of methylated DNA was achieved by fragmentation of the unmethylated DNA strands by the MSREs, whereas methylated DNA remains uncleaved even in the presence of MSREs. Digested DNA samples were amplified in 16 multiplexed PCR reactions amplifying a total of 360 methylation marker candidates (targeting CpG islands and human gene promoters) using biotinylated revere-primers. Pooled amplicons were detected on a targeted DNA-microarray via streptavidin-Cy3. The principle methodology has been published previously (Pulverer, W., et al., Biochimie, 2012. 94(11): p. 2345-52). From the chip-based analyses significant markers were identified applying statistical tests for class comparison and class prediction. From these analyses the best predictive gene sets were used for qPCR confirmation by SYBR Green based qPCR. A p-value of p<0.05 was considered as statistically significant.

### Example 3: Confirmation of markers by MSRE coupled qPCR

Eighteen samples, from which both tumorous and adjacent normal tissue was available, were subjected to a microfluidic high-throughput qPCR (µHT-qPCR) system (Fluidigm's Biomark, San Francisco, USA) for confirmation of differentially methylated gene regions. Prior to the µHT-qPCR experiments, the samples were digested with the same combination of MSREs as mentioned above (3 µl 10x Buffer Tango, 0.3 µl HpaII, 0.3 µl Hin6I, 0.3 µl AciI, 0.3 µl HpyCH4IV, 15 µl DNA with a concentration of 40 ng/µl, 10.8 µl H₂O, final volume: 30 µl, final DNA concentration: 20 ng/µl). The samples were incubated for 16 h at 37 °C, followed by 65 °C for 20 min to deactivate the MSREs. Digests were preamplified using a primer mix consisting of 48 primer pairs (Table 1) at a final concentration of 200 nM of each primer. 6.25 µl of the primer mix, 2.5 µl 10x buffer without MgCl₂, 0.15 U Qiagen HotStarTaq (Hilden, Germany), 1.25 µl DMSO, 2 µl 2 mM dNTP-mix, 7.85 µl H₂O, and 5 µl of the digested DNA (100 ng) were mixed and subjected to PCR amplification: (cycling conditions: 95 °C for 10 min, 14 cycles at 95 °C for 15 s and 65 °C for 4 min). After preamplification the reactions were diluted 1:5 with H₂O. For the readout of the enriched, undigested (and thus methylated) targets prepared nanoliter qPCR was conducted in a Biomark system (Fluidigm, San Francisco) using 48.48 dynamic qPCR arrays for the simultaneous investigation of 48 samples by 48 qPCR assays. The microfluidic 48.48 arrays was loaded 1) with 6 µl of a sample mix, containing 1.5 µl of the diluted sample from the preamplification, 0.33 µl EvaGreen (Biotium, California, USA), 0.036 µl HotStarTaq Polymerase (Qiagen, Hilden, Germany), 0.6 µl 20x loading reagent (Fluidigm, San Francisco, USA), 0.004 µl ROX, 0.3 µl DMSO, 0.6 µl 10x PCR buffer with 1.5 mM MgCl₂ (Qiagen, Hilden, Germany), 0.48 µl 2 mM dNTP-mix and 2.15 µl H₂O and 2) loaded with 5 µl assay mix containing 2.5 µl assay loading reagent (Fluidigm, San Francisco, USA), 0.25 µl H₂O and 2.25 µl of pooled forward and reverse primer (20 µM each primer). Fluidigm's 48.48 GE Dynamic Arrays were primed and loaded with the sample- and assay-mix according to the manufacturer's protocol. Cycling conditions were as followed: 50 °C for 2 min, 95 °C for 10 min, 35 cycles at 95 °C for 15 s and 65 °C for 1 min.

Table 1: Complete information on the 48 primer pairs included in the validation experiments. The panel contained 6 controls to ensure normalization of the data (* methylated targets; ⁺ target without cut sites for MSREs; ^{#} unmethylated target).

**Table 1:**

| | **Gene Symbol** | **chr. Position** | **target size** | **forward primer SEQ ID** | **forward primer sequence** | **reverse primer SEQ ID** | **reverse primer sequence** |
|---|---|---|---|---|---|---|---|
| **1** | BOLL | chr2:198651373-198651480 | 107 | 1 | cgcggacgccgctctgcacct | 2 | cggagcccgactgggctgaa tggc |
| **2** | CALCA | chr11:14993779-14993879 | 100 | 3 | tcggggctcacctggcgggag | 4 | cccaagtgtcgccgccgctt cc |
| **3** | CD24 | chrY:21154660-21154760 | 100 | 5 | ggcgggcgcaggcaaggtgg | 6 | gcgccgaccagccgggaagg |
| **4** | CDX1 | chr5:149546840-149546941 | 101 | 7 | gtggatgcggcgcagcgtgg | 8 | gggccgcgcccgagcacct |
| **5** | CHFR | chr12:133464631-133464739 | 108 | 9 | tggacgcggggccacgtcttg | 10 | acgtgaacgccgccggat- tccaa |
| **6** | CLIC4 | chr1:25070859-25070979 | 120 | 11 | gcctgaaacacagtaaggttt | 12 | cgatctcctgacctcgtg |
| **7** | CXADR | chr21:18884389-18884479 | 90 | 13 | cctgcccagttactgccccac- tccg | 14 | tgccgcgacagttcca- taacagaaatgaa |
| **8** | DAPK1 | chr9:90113220-90113325 | 105 | 15 | gtggcgccggcccgaccagg | 16 | ccgcccgggggagcaaggga |
| **9** | DCC | chr18:49866565-49866657 | 92 | 17 | cgcacaccgctggcggacacc | 18 | cgagagccgaggg- gacccaggga |
| **10** | ESR1 | chr6:152129384-152129482 | 98 | 19 | cgccgccgcagctgtcgcctt | 20 | gccggcctcgcgcaccgtgt |
| **11** | FMR1 | chrX:146993399-146993481 | 82 | 21 | ttcagtgtttacacccgcagc | 22 | ctccaccggaagtgaaaccg |
| **12** | GATA4 | chr8:11562581-11562686 | 105 | 23 | gccggggtcgcggactgcca | 24 | cgcgctgccccagggat- tcca |
| **13** | GDNF | chr5:37839844-37839948 | 104 | 25 | gggcccccgcacccccagaa | 26 | cccgcgcagccccagccaag |
| **14** | H19* | chr11:2018057-2018158 | 101 | 27 | tgggccgcagtgcctcgtggg | 28 | gggcgaagcggccacgggag |
| **15** | HLA-G | chr6:29796001-29796100 | 99 | 29 | gggaagaggagacacggaacac- ca | 30 | tactcac- tggcctcgctctggttg |
| **16** | IL1B | chr2:113595391-113595483 | 92 | 31 | ctcggccccctgcaagttcc | 32 | cgtggcggcaggtgcctgta |
| **17** | IRF4⁺ | chr6:925744-925844 | 100 | 33 | aaaggccccttgg- gaaaccctcacc | 34 | ggaaggcaaaggctgtgaca ggcatcttt |
| **18** | JUB⁺ | chr14:23447607-23447708 | 101 | 35 | ggcattgctctgccca- tagatgcctttg | 36 | ggaatccctggttttgac- ctgggga |
| **19** | MSH4 | chr1:76262522-76262631 | 109 | 37 | gctgagcctgcctgcgcctg | 38 | tgagcgacccctccag- tagcccaa |
| **20** | MYOD1 | chr11:17741576-17741673 | 97 | 39 | cgcccagcgggcaccaccag | 40 | gtggcggccttgcggcggtc |
| **21** | NKX2-1 | chr14:36988068-36988172 | 104 | 41 | gctgccgggccgccctccct | 42 | actgcggggccgcgggctg |
| **22** | PENK | chr8:57358637-57358742 | 105 | 43 | ctttctggggccccgccctccc | 44 | cctcgcgttgggggcgaccg |
| **23** | PITX2 | chr4:111544363-111544471 | 108 | 45 | acgtggctccgcaccaatcag- gacg | 46 | cccagtgcgcacggcgaggc |
| **24** | PITX2 | chr4:111555217-111555308 | 91 | 47 | gcgggtgcggggatcggggt | 48 | caggccagaggcaggcccg- cag |
| **25** | PTGS2 | chr1:186649383-186649484 | 101 | 49 | cgccaggaccgcgcacagca | 50 | cacagccagacgccctcag- acagca |
| **26** | RARB | chr3:25469391-25469493 | 102 | 51 | cgggcgcaggcggaacaccg | 52 | cgggac- gctgctcctggctcacgtt |
| **27** | RHOXF1 | chrX:119249518-119249626 | 108 | 53 | ggcgggggctgcggctgct | 54 | ccctgagggcggtgtgaac- cacga |
| **28** | S100A2 | chr1:153541149-153541259 | 110 | 55 | cggccccgcgggattttgcc | 56 | ccagagggtctggcg- gacaagaaaggg |
| **29** | S100A8 | chr1:153363706-153363808 | 102 | 57 | gggtggggaggtaggagaagcg- gaaa | 58 | tgccagcagcccagaaaaa- gagccc |
| **30** | SALL3 | chr18:76740782-76740882 | 100 | 59 | cccgccgcccggccagcttt | 60 | caggctgcgtcaac- gcgctggg |
| **31** | SER-PINB2 | chr18:61553509-61553616 | 107 | 61 | tgtt- gagctgtcaatgtgtggttaaaa a | 62 | tcaggcaatccgcccacc |
| **32** | SEZ6L | chr22:26565613-26565713 | 100 | 63 | ctccgcgccccctgcagcca | 64 | cgccgggctccccaggag- cag |
| **33** | SFRP2 | chr4:154710061-154710169 | 108 | 65 | cccgggcccgctctcttcgc | 66 | cgcctcgcccgcgctgtcct |
| **34** | SNRPN* | chr15:25200422-25200527 | 105 | 67 | gtcccccatccgcccccaactg | 68 | cccactgcggttaccccg- catgctc |
| **35** | SPARC | chr5:151066401-151066501 | 100 | 69 | aggctgggcgggggtcacaca | 70 | cccctccacattcccgcggt cc |
| **36** | SRGN | chr10:70847793-70847898 | 105 | 71 | cgtgtgttctgggcagggttt- gagg | 72 | gctctcccagctgcac- gccaa |
| **37** | TBP^{#} | chr6:170862257-170862358 | 101 | 73 | ggcccgcggctctgtgcg | 74 | gtgtcggatccgcaggcg- cag |
| **38** | TCEB2^{#} | chr16:2827419-2827521 | 102 | 75 | cgtgcggccgccatcccgac | 76 | gcccgccggaccgcaccaac |
| **39** | TFPI2 | chr7:93519961-93520058 | 97 | 77 | gacagccccagggggcgagcg | 78 | cgcgcacctcctcccgccag |
| **40** | THBD | chr20:23031049-23031143 | 94 | 79 | cgggtggggaagtcgcgggga | 80 | ccctacccggcgccgcag- caa |
| **41** | TJP2 | chr9:71789361-71789467 | 106 | 81 | tgtgccgcgcggttgggagg | 82 | cagcttcctacggcg- catcgggga |
| **42** | TMEFF2 | chr2:193059555-193059661 | 106 | 83 | tccaggggcagacgagtg- gagccc | 84 | ccagagggatgcgggcgg- caga |
| **43** | TP53 | chr17:7591675-7591783 | 108 | 85 | ccgcgggttccgtgggtcgc | 86 | caccgcgggtcgctac- gggcct |
| **44** | TWIST1 | chr7:19157180-19157280 | 100 | 87 | ccaacggctggacgcacacccc | 88 | ccaagtctg- cagctctcgcccaactccc |
| **45** | WT1 | chr11:32455406-32455516 | 110 | 89 | tgtcccagctgagccccgcgtt | 90 | gcgggaccaggttctaccg- caggc |
| **46** | XIST | chrX:73071028-73071136 | 108 | 91 | aggcggcaaaacccgccatc | 92 | tgccgcagggacaatatgg- ca |
| **47** | ZNF256 | chr19:58459043-58459152 | 109 | 93 | cgaccgccacaaggaggacaac- gga | 94 | tgcgttgggcgacggcgac- ctt |
| **48** | ZNF502 | chr3:44754103-44754204 | 101 | 95 | agctcgctgctgacgcactaccg | 96 | ggcgcaggcgctcccaga |

### Example 4: Bioinformatics and statistics

The microarrays were scanned on an Axon 4000B microarray scanner. Data derived from the screening experiments were quantile-normalized and differentially methylation between the sample groups was identified using a random-variance t-test and different classification algorithms implemented in BRB Array tools (BRB-AT; Version 4.2.1; https://linus.nci.nih.gov/BRB-ArrayTools.html) and in R statistical software (Version 2.14.2). For the analyses an adjusted p-value (Benjamini-Hochberg) <0.05 was considered significant (Benjamini, Y. and Y. Hochberg, Journal of the Royal Statistical Society Series B-Methodological, 1995. 57(1): p. 289-300).

Classification of samples into tumour and control groups was done using the measured signal intensities from the methylation array as features in the model. Missing values were imputed using KNN-Impute (Troyanskaya, O., et al., Bioinformatics, 2001. 17(6): p. 520-5.). The different prediction algorithms implemented in BRB-AT (*Compound Covariate Predictor; Diagonal Linear Discriminant Analysis; k-Nearest-Neighbour; Nearest Centroid; Support Vector Machines, Bayesian Compound Covariate Predictor; Prediction analysis of microarrays* (*PAM*)) was used to train the data to identify a highly sensitive and specific set of biomarkers. The results were cross-validated in leave-one-out-crossvalidation (loocv) to estimate the performance of the established models. The identified genomic regions from the screening experiments, either being part of one of the classifiers or genomic regions with high differential methylation between the sample groups were confirmed by qPCR (Biomark, Fluidigm, San Francisco).

Raw cp-values and tm-values were exported to text files for further analysis. Samples with missing values were understood as completely digested, and the respective cp-value was set to 40, which corresponds to the 100% unmethylated state of the loci.

### Example 5: Elucidation of DNA methylation changes in cancer vs. control tissue

22 fresh frozen rectal cancer DNAs together with 8 control samples derived from PBMCs were subjected to microarray based methylation analyses. Class comparison (tumor tissue vs. PBMCs) using Student's t-test identified 53 (14.7%) out of 360 genes with differentially methylated 5'UTR gene regions (DMR) between the tumor tissue and PBMCs. The log2 fold change of the microarray mean signal intensities between tumor tissues and PBMCs were calculated for each gene to characterize the grade of differential methylation. Eight different prediction algorithms were applied to the data. The classification algorithms allowed an up to 100% correct identification of each sample.

The top 22 DMRs indicating differential methylation between carcinoma and PBMCs at a high level of significance (p<0.001) or contributing to one of the classification rules were employed for further validation experiments on Fluidigm's Biomark.

### Example 6: Validation of the differentially methylated regions (DMR)

In order to validate the results from the microarray screen, the 48 chosen targets (Table 1; 22 DMRs from the screen, 20 cancer associated genes, 6 control genes) and 48 DNA samples (18 tumor samples from the screen plus 18 tumor samples from the adjacent tissue of the patients and 12 PBMC samples), were subjected to Fluidigm's Biomark (48.48 chip). We aimed to elucidate DNA methylation markers for distinguishing between **1)** the tumor tissue (n=18) vs. PBMCs (n=12), **2)** tumor tissue (n=18) vs. adjacent tissue (n=18), **3)** tumor tissue (n=18) vs. adjacent tissue (n=18) and PBMCs (n=12), allowing correct classification of the different samples. A predictor for each classification algorithm was computed and included the calculation of correct classification successes for each predictor.

### Example 7: Differentially methylated regions between tumor tissue samples and PBMC samples

The comparison of the tumor tissue samples with the PBMC samples showed the best classification success, as all eight classification algorithms were able to allocate all samples to the correct group (100% classification success; sensitivity: 1; specificity 1). Predicted probabilities derived from Bayesian Compound Covariate for each PBMC sample was 1 to belong to the class PBMC and for each tumor sample the predicted probability to belong to class PBMC was 0.001. With exception of the PAM classifier, the mean number of necessary genes for each predictor to allocate all samples correctly was 26. For the PAM algorithm the methylation state of the two genes TFPI2 and TJP2 was sufficient to allocate 100% of the samples correctly (sensitivity: 1; specificity: 1). The promoter regions of all 26 genes listed in table 2 were found hypermethylated in the tumorous tissue.

**Table 2: Composition of classifier for tumor tissue vs. PBMC**

| | **Gene Symbol** | **p-value** | **mean intensities in PBMC** | **mean intensities in tumour tissue** | **Fold-change** |
|---|---|---|---|---|---|
| **1** | ESR1 | < 1*10⁻⁰⁷ | 7528603.80 | 792319004.00 | 105.3 |
| **2** | TFPI2 | < 1*10⁻⁰⁷ | 3.68 | 1623538.16 | 434782.6 |
| **3** | WT1 | < 1*10⁻⁰⁷ | 18709939.90 | 986310917.00 | 52.6 |
| **4** | TMEFF2 | < 1*10⁻⁰⁷ | 2665343.21 | 274613795.00 | 103.1 |
| **5** | PENK | < 1*10⁻⁰⁷ | 72682.19 | 19433196.90 | 270.3 |
| **6** | MYOD1 | < 1*10⁻⁰⁷ | 10704363.60 | 238161264.00 | 22.2 |
| **7** | TWIST1 | < 1*10⁻⁰⁷ | 16854218.80 | 728256573.00 | 43.5 |
| **8** | DCC | < 1*10⁻⁰⁷ | 3.73 | 11908.93 | 3225.8 |
| **9** | PTGS2 | < 1*10⁻⁰⁷ | 9.76 | 46576.73 | 4761.9 |
| **10** | TJP2 | < 1*10⁻⁰⁷ | 3010.66 | 118657427.00 | 40000.0 |
| **11** | SPARC | 4.0*10⁻⁰⁷ | 1935341.25 | 80209027.00 | 41.7 |
| **12** | PITX2 | 4.0*10⁻⁰⁷ | 1464.81 | 418420.69 | 285.7 |
| **13** | SEZ6L | 1.2*10⁻⁰⁶ | 325.53 | 5617521.35 | 17241.4 |
| **14** | TP53 | 1.4*10⁻⁰⁶ | 1.01 | 1512.22 | 1492.5 |
| **15** | GDNF | 1.5*10⁻⁰⁶ | 47851.23 | 13899432.30 | 294.1 |
| **16** | CDX1 | 1.5*10⁻⁰⁶ | 1953642.36 | 15434192.80 | 7.7 |
| **17** | CLIC4 | 3.4*10⁻⁰⁶ | 2.06 | 2974.07 | 1449.3 |
| **18** | SFRP2 | 3.1*10⁻⁰⁵ | 10019.02 | 52535530.60 | 5263.2 |
| **19** | HLA-G | 3.8*10⁻⁰⁵ | 1237163557.00 | 3459870339.00 | 2.8 |
| **20** | GATA4 | 7.9*10⁻⁰⁵ | 4769897.02 | 37776934.00 | 7.7 |
| **21** | BOLL | 9.8*10⁻⁰⁵ | 297905.44 | 39469517.60 | 133.3 |
| **22** | THBD | 1.7*10⁻⁰⁴ | 5448.29 | 992349.42 | 181.8 |
| **23** | RARB | 9.3*10⁻⁰⁴ | 1.01 | 139.38 | 138.9 |
| **24** | NKX2-1 | 0.007 | 1232763.85 | 8183239.26 | 6.7 |
| **25** | SALL3 | 0.026 | 1.01 | 19.76 | 19.6 |
| **26** | JUB | 0.44 | 2606536030.00 | 3811739113.00 | 1.5 |

### Example 8: Differentially methylated regions between tumor tissue samples and the adjacent tissue

Although the tissue resection sites between tumor tissue and adjacent tissue were in close proximity a distinct methylation pattern has been identified in both groups. In fact, the correct classification rates were inferior compared to the success rates of the tumor tissue vs. PBMC, but the PAM algorithm still identifies 100% of the samples correctly, followed by the Bayesian Compound covariate Predictor (97%), the Diagonal Linear Discriminant Predictor (94%) and the Support Vector Machines (94%). However, the Bayesian Compound Covariate Predictor left 7 samples unclassified. These unclassified samples were excluded in the computation of correct classification rates for the Bayesian Compound Covariate Predictor. Classification success of the remaining 4 predictors were between 75% and 89% (Nearest Centroid 75%; 3-NearestNeighbors and Compound Covariate Predictor 86%; Diagonal Linear Discriminant Analysis 89%). Detailed information can be found in the supplementary appendix S2. Fourteen genes were necessary to achieve the above mentioned classification results, all with p-values <0.01 (Table 3). Depending on the applied predictor, sensitivities between 0.78 (3-Nearest Neighbors/Bayesian Compound covariate) and 0.89 (Diagonal Linear Discriminant/Support Vector Machine) were achieved. The specificities were between 0.72 (Nearest Centroid) and 0.89 (Diagonal Linear Discriminant/Support Vector Machine). Again, the PAM algorithm allowed a 100% correct classification of the samples with a decreased number of genes in the classifier compared to the other predictors. The classifier contains four genes and including the promoter regions of TMEFF2, TWIST1, PITX2 and TFPI2.

**Table 3: Composition of classifier for tumor tissue vs. adjacent tissue**

| | **Gene Symbol** | **p-value** | **mean intensities in adjacent tissue** | **mean intensities in tumor tissue** | **Fold-change** |
|---|---|---|---|---|---|
| **1** | TMEFF2 | < 1*10⁻⁷ | 16256337.53 | 274613795.34 | 16.9 |
| **2** | PITX2 | < 1*10⁻⁷ | 23409.84 | 418420.69 | 17.9 |
| **3** | TWIST1 | < 1*10⁻⁷ | 29765758.97 | 728256572.64 | 24.4 |
| **4** | ESR1 | < 1*10⁻⁷ | 67611145.96 | 792319004.11 | 11.8 |
| **5** | BOLL | 2*10⁻⁷ | 2933962.02 | 39469517.62 | 13.5 |
| **6** | TFPI2 | 2.3*10⁻⁶ | 24935.04 | 1623538.16 | 66.7 |
| **7** | WT1 | 3.9*10⁻⁶ | 176629323.65 | 986310917.02 | 5.6 |
| **8** | GDNF | 6.6*10⁻⁴ | 3027022.36 | 13899432.25 | 4.5 |
| **9** | HLA-G | 0.0015 | 1819166436.16 | 3459870339.43 | 1.9 |
| **10** | PENK | 0.0027 | 5835549.75 | 19433196.93 | 3.3 |
| **11** | SEZ6L | 0.0032 | 653357.53 | 5617521.35 | 8.3 |
| **12** | SFRP2 | 0.0063 | 2590147.11 | 52535530.57 | 20.4 |
| **13** | TCEB2 | 0.0086 | 31.06 | 4634.79 | 149.3 |
| **14** | RARB | 0.0091 | 3.82 | 139.38 | 37.0 |

### Example 9: Differentially methylated regions between tumor tissue vs. adjacent tissue and PBMCs

Finally, a classifier to distinguish the tumor tissue from the rest of the samples (adjacent tissue and PBMCs) was calculated. The correct classification rates were comparable to the discrimination between tumor tissue and adjacent tissue, although a higher number of genes included in the classifiers was necessary to do the discrimination with high confidence. All eight classifier showed correct classification rates between 79% (Nearest Centroid) and 98% (PAM). The Compound Covariate Predictor were able to allocate 83% of the sample correct, 1-Nearest Neighbor and 3-Nearest Neighbor, both identified 85% correct, the Diagonal Linear Discriminant has a success rate of 90%, Support Vector Machines had 94% and the Bayesian Compound Covariate Predictor allows the correct classification of 97% of the sample. However, again the Bayesian Compound Covariate Predictor left some (n=8) samples unclassified. Detailed information can be found in the supplementary appendix S3. Twenty-four genes were necessary to achieve the above mentioned classification results, all with p-values <0.05 (Table 4)Table 4. Depending on the applied predictor, sensitivities between 0.72 (1-Nearest Neighbors/3-Nearest Neighbors/Bayesian Compound covariate) and 1 (Diagonal Linear Discriminant/Support Vector Machine) were achieved. The specificities were between 0.72 (1-Nearest Neighbors/3-Nearest Neighbors/Bayesian Compound Covariate) and 0.94 (Compound Covariate Predictor). For the PAM algorithm a set of 10 genes was sufficient to allocate 98% of the samples correct. Also in the discrimination of tumor tissue vs. all other samples (PBMC plus adjacent tissue) a diminished set of gens is enough for the PAM algorithm. Genes contributing to the PAM classifier were TFPI2, TMEFF2, TWIST1, ESR1, PITX2, DCC, WT1, SEZ6L, BOLL and SFRP2.

**Table 4: Composition of classifier for tumor tissue vs. adjacent tissue plus PBMCs**

| | | **p-value** | **mean of intensities in adjacent tissue and PBMCs** | **mean of intensities in tumor tissue** | **Fold-change** |
|---|---|---|---|---|---|
| **1** | TWIST1 | < 1*10⁻⁰⁷ | 23709021.31 | 728256572.64 | 30.30 |
| **2** | TMEFF2 | < 1*10⁻⁰⁷ | 7887055.21 | 274613795.34 | 34.48 |
| **3** | ESR1 | < 1*10⁻⁰⁷ | 28099381.15 | 792319004.11 | 28.57 |
| **4** | WT1 | < 1*10⁻⁰⁷ | 71955938.20 | 986310917.02 | 13.70 |
| **5** | PITX2 | 2.00*10⁻⁰⁷ | 7725.95 | 418420.69 | 55.56 |
| **6** | TFPI2 | 2.00*10⁻⁰⁷ | 731.74 | 1623538.16 | 2222.22 |
| **7** | HLA-G | 4.50*10⁻⁰⁶ | 1559173009.28 | 3459870339.43 | 2.22 |
| **8** | PENK | 1.91*10⁻⁰⁵ | 1009764.91 | 19433196.93 | 19.23 |
| **9** | BOLL | 2.56*10⁻⁰⁵ | 1175179.12 | 39469517.62 | 33.33 |
| **10** | DCC | 8.04*10⁻⁰⁵ | 77.93 | 11908.93 | 153.85 |
| **11** | TP53 | 8.77*10⁻⁰⁵ | 9.66 | 1512.22 | 156.25 |
| **12** | GATA4 | 1.37*10⁻⁰⁴ | 9538475.52 | 37776934.04 | 4.00 |
| **13** | GDNF | 1.62*10⁻⁰⁴ | 576191.19 | 13899432.25 | 24.39 |
| **14** | RARB | 1.78*10⁻⁰⁴ | 2.24 | 139.38 | 62.50 |
| **15** | CLIC4 | 2.07*10⁻⁰⁴ | 22.64 | 2974.07 | 131.58 |
| **16** | SEZ6L | 4.11*10⁻⁰⁴ | 31198.01 | 5617521.35 | 178.57 |
| **17** | SFRP2 | 6.58*10⁻⁰⁴ | 280753.27 | 52535530.57 | 188.68 |
| **18** | PTGS2 | 0.003 | 954.42 | 46576.73 | 50.00 |
| **19** | THBD | 0.004 | 43191.96 | 992349.42 | 22.73 |
| **20** | TJP2 | 0.005 | 1474676.53 | 118657426.72 | 83.33 |
| **21** | SPARC | 0.011 | 17816251.06 | 80209027.02 | 4.55 |
| **22** | TCEB2 | 0.020 | 72.39 | 4634.79 | 62.50 |
| **23** | MYOD1 | 0.022 | 30965125.57 | 238161264.23 | 7.69 |
| **24** | S100A2 | 0.024 | 6345046.25 | 1241558.75 | 0.20 |

### Example 10: Further marker subsets

Additional methylation marker set with high diagnostic power have been identified based on the above tables of highly relevant methylation specific gene markers. It could be shown, that in all cases it is not necessary to use all the marker genes of tables 2-4 given above, but it also reduced set were sufficient of e.g. 1, 2, 3, 4, or 5 genes. Examples are provided as follows:

**Table 5: TFPI2 as single classifier provided the following correct classifications. Samples used in this experiment were cancer tissue vs. blood:**

| **Algorithm** | Support Vecotor Machines | Nearest Centroid | 1-nearest neigbor | 3-nearest neighor | Diagnoal Linear Discriminant Analysis | Compound Covarivariate Predictor | Bayesian Compound Covarivariate Predictor |
|---|---|---|---|---|---|---|---|
| **Correct classification** | 97% | 93% | 97% | 90% | NA | 93 | 96 |

A set consisting of 5 genes (100% Classification success with all algorithms) comprises methylation markers: TFPI2, DCC, PTGS2, TJP2, PITX2. A set consisting of 4 genes (100% Classification success with all algorithms) comprises methylation markers: TFPI2, DCC, PTGS2, TJP2. A set consisting of 3 genes (100% Classification success with all algorithms) comprises methylation markers: TFPI2, DCC, PTGS2. Set consisting of 2 genes (100% Classification success with Compound Covariate Predictor, Support Vector Machines, Bayesian Compound Covariate, 97% correct classification success with Diagonal Linear Discriminant Analysis, k-Nearest Neighbour, Support Vector Machine) comprises methylation markers: TFPI2, PTGS2. Samples used in these experiments were cancer tissue vs. blood.

**Table 6: A set consisting of 10 genes: TMEFF2, PITX2, TWIST1, SFRP2, RARB, ZNF256, SPARC, DAPK1, CALCA, S100A2 achieved the following classification. Samples used in this experiment were cancer tissue vs. normal (adjacent) tissue:**

| **Algorithm** | Compound Covarivariate Predictor | Diagonal Linear Discri-minant Analysis | 1-Nearest Neighbor | 3-Nearest Neighbors | Nearest Cen-troid | Support Vec-tor Machines | Bayesian Compound Covarivariate Predictor |
|---|---|---|---|---|---|---|---|
| **Correct classification** | 97 | 97 | 94 | 92 | 81 | 92 | 97 |

**Table 7: A set consisting of 7 genes: TMEFF2, PITX2, TWIST1, SFRP2, ZNF256, SPARC, DAPK1 achieved the following classification. Samples used in this experiment were cancer tissue vs. normal (adjacent) tissue:**

| **Algorithm** | Compound Covarivariate Predictor | Diagonal Linear Discriminant Analysis | 1-Nearest Neigh bor | 3-Nearest Neighbors | Nearest Centroid | Support Vector Machines | Bayesian Compound Covarivariate Predictor |
|---|---|---|---|---|---|---|---|
| **Correct classification** | 100 | 100 | 94 | 92 | 92 | 94 | 100 |

**Table 8: A set consisting of 5 genes: TMEFF2, PITX2, TWIST1, SPARC, S100A2 achieved the following classification. Samples used in this experiment were cancer tissue vs. normal (adjacent) tissue:**

| **Algorithm** | Compound Covari variate Predictor | Diagonal Linear Discriminant Analysis | 1-Nearest Neighbor | 3-Nearest Neighbors | Nearest Centroid | Support Vector Machines | Bayesian Compound Covarivariate Predictor |
|---|---|---|---|---|---|---|---|
| **Correct classification** | 97 | 97 | 94 | 94 | 97 | 92 | 97 |

**Table 9: A set consisting of 2 genes: TMEFF2, TWIST1 achieved the following classification. Samples used in this experiment were cancer tissue vs. normal (adjacent) tissue:**

| **Algorithm** | Compound Covari variate Predictor | Diagonal Linear Discriminant Analysis | 1-Nearest Neighbor | 3-Nearest Neighbors | Nearest Centroid | Support Vector Machines | Bayesian Compound Covarivariate Predictor |
|---|---|---|---|---|---|---|---|
| **Correct classification** | 97 | 97 | 97 | 94 | 94 | 94 | 97 |

**Table 10: A set consisting of 1 gene: TWIST1 achieved the following classification. Samples used in this experiment were cancer tissue vs. normal (adjacent) tissue:**

| **Algorithm** | Compound Covari variate Predictor | Diagonal Linear Discriminant Analysis | 1-Nearest Neighbor | 3-Nearest Neighbors | Nearest Centroid | Support Vector Machines | Bayesian Compound Covarivariate Predictor |
|---|---|---|---|---|---|---|---|
| **Correct classification** | 97 | 97 | 92 | 92 | 97 | 94 | 97 |

As shown by these examples, suitable marker subsets with any number of diagnostically relevant classification outcomes can be selected from the full list of all markers with ease using statistical methods. This shows that the markers of tables 2-4 are of highest diagnostic relevance, which when combined can achieve remarkable success.

### Example 11: Validation of identified candidate markers in serum samples:

The DNA methylation based biomarkers underwent further experiments using serum samples from an independent sample cohort. For the additionally conducted experiments the same set of biomarkers were used and applied to 20 colon cancer samples and to 20 neoplasia free (healthy control) samples - each group with 10 male and 10 female samples.

The 40 samples were processed under the same conditions as the previous samples. In brief, the samples were digested over night with four different methylation sensitive restriction enzymes (HpaII, Hin6I, AciI, HpyCH4IV) at 37°C. After the overnight incubation the methylation sensitive restriction enzymes were heat inactivated for 20 minutes at 65 °C. The digested DNA samples were subjected to a multiplexed PCR (in the following referred to as "preamplification") to enrich the targets of interest (n=48 [including 6 controls]). The preamplified targets of interest were diluted with H₂O (1:5) and applied to the microfluidic based high throughput qPCR device (Biomark, Fluidigm). The provided cp-values were filtered according to the melting temperature (tm) of the amplicons, meaning that only cp-values for samples with correct tm-values were used for the subsequent calculations. Additionally, the cp-values were normalized using the included control genes.

**Table 11: Best markers found highly differentially methylated in both sample sets, the initially used tissue vs. adjacent tissue or PBMC sample set and the serum sample set.**

| | **initial sample set** | | **serum sample set** | |
|---|---|---|---|---|
| | **p-value** | **Fold-change** | **p-value** | **Fold-change** |
| **GATA4** | 1.81E-02 | 2.50 | 2.53E-03 | 1.25 |
| **WT1** | 3.90E-06 | 5.56 | 2.60E-03 | 1.92 |
| **TFPI2** | 2.30E-06 | 66.67 | 8.79E-03 | 119.05 |
| **SEZ6L** | 3.23E-03 | 8.33 | 1.31E-02 | 2.27 |
| **TMEFF2** | <1.00E-07 | 16.95 | 1.73E-02 | 1.28 |
| **DCC** | 1.40E-02 | 20.00 | 3.11E-02 | 1.79 |
| **BOLL** | 2.00E-07 | 13.51 | 3.23E-02 | 32.26 |
| **PITX2** | <1.00E-07 | 17.86 | 3.90E-02 | 1.52 |
| **GDNF** | 6.60E-04 | 4.55 | 4.45E-02 | 45.45 |

The following tables show different biomarker compositions, used to discriminate malign from neoplasia free samples:

**Table 12: TMEFF2 as single classifier:**

| **Algorithm** | Compound Covariate Predictor | Diagnoal Linear Discriminant Analysis | 1-nearest neigbor | 3-nearest neighor | Nearest Centroid | Support Vector Machines | Bayesian Compound Covariate Predictor |
|---|---|---|---|---|---|---|---|
| **Correct classification (%)** | 71 | 71 | 63 | 58 | 71 | 66 | 67 |

**Table 13: Set consisting of 2 genes: TMEFF2, TWIST1**

| **Algorithm** | Compound Covariate Predictor | Diagnoal Linear Discriminant Analysis | 1-nearest neigbor | 3-nearest neighor | Nearest Centroid | Support Vector Machines | Bayesian Compound Covariate Predictor |
|---|---|---|---|---|---|---|---|
| **Correct classification (%)** | 71 | 71 | 63 | 66 | 71 | 68 | 50 |

**Table 14: Set consisting of 3 genes TMEFF2, PITX2, TWIST1**

| **Algorithm** | Compound Covariate Predictor | Diagnoal Linear Discriminant Analysis | 1-nearest neigbor | 3-nearest neighor | Nearest Centroid | Support Vector Machines | Bayesian Compound Covariate Predictor |
|---|---|---|---|---|---|---|---|
| **Correct classification (%)** | 55 | 61 | 53 | 61 | 55 | 66 | 83 |

**Table 15: Set consisting of 4 genes: TMEFF2, TWIST1, PTX2, S100A2**

| **Algorithm** | Compound Covariate Predictor | Diagnoal Linear Discriminant Analysis | 1-nearest neigbor | 3-nearest neighor | Nearest Centroid | Support Vector Machines | Bayesian Compound Covariate Predictor |
|---|---|---|---|---|---|---|---|
| **Correct classification (%)** | 55 | 66 | 61 | 71 | 55 | 68 | 71 |

**Table 16: Set consisting of 7 genes: DAPK1, TMEFF2, PITX2, S100A2, ZNF256, RARB, SFRP2**

| **Algorithm** | Compound Covariate Predictor | Diagnoal Linear Discriminant Analysis | 1-nearest neigbor | 3-nearest neighor | Nearest Centroid | Support Vector Machines | Bayesian Compound Covariate Predictor |
|---|---|---|---|---|---|---|---|
| **Correct classification (%)** | 68 | 61 | 61 | 63 | 74 | 63 | 93 |

**Table 17: Set consisting of 10 genes: DAPK1, TMEFF2, PITX2, S100A2, SPARC, CALCA, TWIST1, ZNF256, RARB, SFRP2**

| **Algorithm** | Compound Covariate Predictor | Diagnoal Linear Discriminant Analysis | 1-nearest neigbor | 3-nearest neighor | Nearest Centroid | Support Vector Machines | Bayesian Compound Covariate Predictor |
|---|---|---|---|---|---|---|---|
| **Correct classification (%)** | 68 | 58 | 68 | 71 | 68 | 61 | 93 |

### Experimental Summary

The presented study focused on the screening and validation of gene panels for the correct identification of CRC. As CRC is the third most common cause for cancer related deaths, the early identification of the disease is of utmost interest. CRC related deaths could be dramatically decreased by early identification of the disease, at the best when the disease is in a premalignant state when medical treatment is highly effective. The onset of epigenetic abnormalities takes place before tumor formation and is amongst others a prerequisite for tumorigenesis. Thus makes epigenetic tumor causing events the ideal source for the development of biomarkers. Different types of epigenetic biomarkers exist, like miRNAs and histone modifications, but the most promising candidate for biomarker development is in our opinion the change in the DNA methylation pattern. Methylation of the cytosine is a stable modification of the DNA, the DNA itself is more or less relatively resistant against extern exposure to different influences and in the lab easy to handle compared to e.g. mRNA or miRNA. Therefore we aimed to identify gene marker panel based on DNA methylation analysis to develop a tool for the early diagnostics of CRC.

The targeted screen for methylation marker candidates which was performed in 22 rectal tumor samples and 8 PBMCs identified 53 possible candidates for the marker panel. The most of the genes were found hypermethylated in the tumorous samples. This result is in accordance with the literature, as the microarray contains only targets located in the 5'UTR regions of cancer associated genes and those regions are most likely are hypermethylated in tumor tissue. The top differentially methylated regions identified by random variance t-test were in most instances also part of the 8 different classification algorithms which were applied to the microarray data.

Since the methylation structure of the predictive panel is also divers from the methylation structure of PBMCs, the use of sera for the detection of cancerous gene methylation is possible.

The application of biomarkers for early identification of therapy success is crucial to allow a switch to another therapy regimen in case of therapy failure.

The invention of biomarkers, accurately detecting patients with high risk for CRC could massively contribute to an improved treatment of patients.

## Claims

1. Method of identifying a colorectal cancer in a sample comprising DNA from a subject, determining the methylation status of two or more genes comprising TFPI2 and PTGS2 and comparing the methylation status with a control sample, thereby identifying colorectal cancer DNA in the sample.

2. The method of claim 1, **characterized in that** the one or more genes comprise the markers of one of the following groups:
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 and WT1;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 and JUB;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 and S100A2;
h) TFPI2, DCC, PTGS2, TJP2, PITX2;
i) TFPI2, DCC, PTGS2, TJP2;
j) TFPI2, DCC, PTGS2;
or a group of markers comprising at least 50%, preferably at least 60%, at least 70%, at least 80%, at least 90%, 100% of the genes of anyone of the above groups a), b), f), h), i), j), wherein, for a given group, at least the 2 genes selected from TFPI2 and PTGS2 are represented.

3. The method of claim 1 or 2, **characterized in that** the step of determining the methylation status comprises a methylation specific PCR analysis, methylation specific digestion analysis, preferably with hybridization analysis to non-digested or digested fragments, or PCR amplification analysis of non-digested or digested fragments, or bisulfite deamination followed by identification of methylated C changes, preferably by PCR or hybridization.

4. The method of any one of claims 1 to 3, comprising comparing the methylation status with the status of a confirmed colorectal cancer positive and/or negative state.

5. The method of any one of claims 1 to 4, **characterized in that** the sample of the subject is a colorectal tissue sample, preferably of a colorectal polyp, or a blood or serum sample, especially a blood, plasma or serum sample taken from the circulatory system of the subject.

6. The method of any one of claims 1 to 5, **characterized in that** the control sample is a colorectal tissue sample, a sample adjacent to a colorectal polyp or cancer tissue, or a blood, plasma or serum sample, especially a sample comprising blood cells, such as PBMCs.

7. The method of any one of claims 1 to 6, **characterized in that** the methylation status of said genes is determined in an upstream region of the open reading frame of the marker genes, in particular a promoter region; or in a) a genetic locus defined by the primer pairs of SEQ ID NOs: 1 and 2, 3 and 4, 5 and 6, 7 and 8, 9 and 10, 11 and 12, 13 and 14, 15 and 16, 17 and 18, 19 and 20, 21 and 22, 23 and 24, 25 and 26, 27 and 28, 29 and 30, 31 and 32, 33 and 34, 35 and 36, 37 and 38, 39 and 40, 41 and 42, 43 and 44, 45 and 46, 47 and 48, 49 and 50, 51 and 52, 53 and 54, 55 and 56, 57 and 58, 59 and 60, 61 and 62, 63 and 64, 65 and 66, 67 and 68, 69 and 70, 71 and 72, 73 and 74, 75 and 76, 77 and 78, 79 and 80, 81 and 82, 83 and 84, 85 and 86, 87 and 88, 89 and 90, 91 and 92, 93 and 94, 95 and 96, and/or b) the chromosomal locus as identified in table 1; or c) a locus within at most 1000 nucleotides in length distanced from said locus a) or b).

8. A set of nucleic acid primers or hybridization probes being specific for a potentially methylated region of marker genes being suitable to diagnose or predict colorectal cancer, with the set comprising probes or primers for genes:
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 and WT1;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 and JUB;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 and S100A2;
h) TFPI2, DCC, PTGS2, TJP2, PITX2;
i) TFPI2, DCC, PTGS2, TJP2;
j) TFPI2, DCC, PTGS2;
k) TFPI2, PTGS2;
or probes or primers for at least 50%, preferably at least 60%, at least 70%, at least 80%, at least 90%, 100% of the genes of anyone of the above a), b), f), h), i), j), with the proviso that at least the 2 genes selected from TFPI2 and PTGS2 are represented,
and any of these sets comprises not more than 350 probes or primers.

9. Set according to claim 8, **characterized in that** the primer pairs and probes are specific for a methylated upstream region of the open reading frame of the marker genes, in particular a promoter region; or specific for a) a genetic locus defined by the primer pairs of SEQ ID NOs: 1 and 2, 3 and 4, 5 and 6, 7 and 8, 9 and 10, 11 and 12, 13 and 14, 15 and 16, 17 and 18, 19 and 20, 21 and 22, 23 and 24, 25 and 26, 27 and 28, 29 and 30, 31 and 32, 33 and 34, 35 and 36, 37 and 38, 39 and 40, 41 and 42, 43 and 44, 45 and 46, 47 and 48, 49 and 50, 51 and 52, 53 and 54, 55 and 56, 57 and 58, 59 and 60, 61 and 62, 63 and 64, 65 and 66, 67 and 68, 69 and 70, 71 and 72, 73 and 74, 75 and 76, 77 and 78, 79 and 80, 81 and 82, 83 and 84, 85 and 86, 87 and 88, 89 and 90, 91 and 92, 93 and 94, 95 and 96 and/or b) the chromosomal locus as identified in table 1; or c) a locus within at most 1000 nucleotides in length distanced from said locus a) or b) .

10. Set according to claim 9, **characterized in that** the probes or primers are of SEQ ID NOs 1 to 96.

11. Set according to any one of claims 8 to 10, **characterized in that** the set is provided in a kit together with a methylation specific restriction enzyme and/or a reagent for bisulfite nucleotide deamination; and/or wherein the set comprises probes on a microarray.

## Patentansprüche

1. Verfahren zur Identifizierung von Kolorektalkrebs in einer Probe, umfassend DNA von einem Subjekt, Bestimmung des Methylierungsstatus von zwei oder mehreren Genen, umfassend TFPI2 und PTGS2, und Vergleichung des Methylierungsstatus mit einer Kontrollprobe, dabei Identifizierung von Kolorektalkrebs -DNA in der Probe.

2. Verfahren nach Anspruch 1, **dadurch gekennzeichnet, dass** die ein oder mehreren Gene die Marker aus einer der folgenden Gruppen umfassen:
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 und WT1;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 und JUB;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 und S100A2;
h) TFPI2, DCC, PTGS2, TJP2, PITX2;
i) TFPI2, DCC, PTGS2, TJP2;
j) TFPI2, DCC, PTGS2;
oder einer Gruppe von Markern, umfassend mindestens 50%, vorzugsweise mindestens 60%, mindestens 70%, mindestens 80%, mindestens 90%, 100% der Gene aus einer der obigen Gruppen a), b), f), h), i), j), wobei, für eine gegebene Gruppe, zumindest die 2 Gene, ausgewählt aus TFPI2 und PTGS2, vertreten sind.

3. Verfahren nach Anspruch 1 oder 2, **dadurch gekennzeichnet, dass** der Schritt des Bestimmens des Methylierungsstatus eine methylierungsspezifische PCR-Analyse, methylierungsspezifische Verdauanalyse, vorzugsweise mit Hybridisationsanalyse an nicht-verdaute oder verdaute Fragmente, oder PCR-Amplifikationsanalyse von nichtverdauten oder verdauten Fragmenten, oder Bisulfit-Desaminierung, gefolgt von Identifizierung der Veränderungen von methyliertem C, vorzugsweise durch PCR oder Hybridisation, umfasst.

4. Verfahren nach einem der Ansprüche 1 bis 3, umfassend das Vergleichen des Methylierungsstatus mit dem Status eines bestätigten Kolorektalkrebs-positiven und/oder -negativen Status.

5. Verfahren nach einem der Ansprüche 1 bis 4, **dadurch gekennzeichnet, dass** die Probe von dem Subjekt eine Kolorektalgewebeprobe, vorzugsweise ein Darmpolyp, oder eine Blut- oder Serumprobe, insbesondere eine Blut-, Plasma- oder Serumprobe ist, die aus dem Kreislaufsystem des Subjekts entnommen ist.

6. Verfahren nach einem der Ansprüche 1 bis 5, **dadurch gekennzeichnet, dass** die Kontrollprobe eine Kolorektalgewebeprobe, eine Probe angrenzend an einen Darmpolyp oder ein Krebsgewebe, oder eine Blut-, Plasma- oder Serumprobe, insbesondere eine Probe, die Blutzellen, wie PBMCs, umfasst, ist.

7. Verfahren nach einem der Ansprüche 1 bis 6, **dadurch gekennzeichnet, dass** der Methylierungsstatus dieser Gene in einer stromaufwärts gelegenen Region des offenen Leserahmens der Markergene, insbesondere einer Promotorregion; oder in a) einem genetischen Locus, der durch die Primerpaare der SEQ ID NRn: 1 und 2, 3 und 4, 5 und 6, 7 und 8, 9 und 10, 11 und 12, 13 und 14, 15 und 16, 17 und 18, 19 und 20, 21 und 22, 23 und 24, 25 und 26, 27 und 28, 29 und 30, 31 und 32, 33 und 34, 35 und 36, 37 und 38, 39 und 40, 41 und 42, 43 und 44, 45 und 46, 47 und 48, 49 und 50, 51 und 52, 53 und 54, 55 und 56, 57 und 58, 59 und 60, 61 und 62, 63 und 64, 65 und 66, 67 und 68, 69 und 70, 71 und 72, 73 und 74, 75 und 76, 77 und 78, 79 und 80, 81 und 82, 83 und 84, 85 und 86, 87 und 88, 89 und 90, 91 und 92, 93 und 94, 95 und 96, definiert ist, und/oder b) dem chromosomalen Locus, wie in Tabelle 1 identifiziert; oder c) einem Locus innerhalb eines Abstands von höchstens 1000 Nukleotiden Länge von dem Locus a) oder b), bestimmt wird.

8. Set von Nukleinsäureprimern oder Hybridisationssonden, das für eine potenziell methylierte Region von Markergenen, die zur Diagnose oder Prognose von Kolorektalkrebs geeignet sind, spezifisch ist, wobei das Set Sonden oder Primer für folgende Gene umfasst:
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 und WT1;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 und JUB;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 und S100A2;
h) TFPI2, DCC, PTGS2, TJP2, PITX2;
i) TFPI2, DCC, PTGS2, TJP2;
j) TFPI2, DCC, PTGS2;
k) TFPI2, PTGS2;
oder Sonden oder Primer für mindestens 50%, vorzugsweise mindestens 60%, mindestens 70%, mindestens 80%, mindestens 90%, 100% der Gene von einem der obigen a), b), f), h), i), j), mit der Maßgabe dass zumindest die 2 Gene, ausgewählt aus TFPI2 und PTGS2, vertreten sind,
und jedes dieser Sets nicht mehr als 350 Sonden oder Primer umfasst.

9. Set gemäß Anspruch 8, **dadurch gekennzeichnet, dass** die Primerpaare und Sonden für eine methylierte stromaufwärts gelegene Region des offenen Leserahmens der Markergene, insbesondere eine Promotorregion, spezifisch sind; oder für a) einen genetischen Locus, der durch die Primerpaare der SEQ ID NRn: 1 und 2, 3 und 4, 5 und 6, 7 und 8, 9 und 10, 11 und 12, 13 und 14, 15 und 16, 17 und 18, 19 und 20, 21 und 22, 23 und 24, 25 und 26, 27 und 28, 29 und 30, 31 und 32, 33 und 34, 35 und 36, 37 und 38, 39 und 40, 41 und 42, 43 und 44, 45 und 46, 47 und 48, 49 und 50, 51 und 52, 53 und 54, 55 und 56, 57 und 58, 59 und 60, 61 und 62, 63 und 64, 65 und 66, 67 und 68, 69 und 70, 71 und 72, 73 und 74, 75 und 76, 77 und 78, 79 und 80, 81 und 82, 83 und 84, 85 und 86, 87 und 88, 89 und 90, 91 und 92, 93 und 94, 95 und 96, und/oder b) den chromosomalen Locus, wie in Tabelle 1 identifiziert; oder c) einen Locus innerhalb eines Abstands von höchstens 1000 Nukleotiden Länge von dem Locus a) oder b), spezifisch sind.

10. Set gemäß Anspruch 9, **dadurch gekennzeichnet, dass** die Sonden oder Primer von SEQ ID NRn: 1 bis 96 sind.

11. Set gemäß einem der Ansprüche 8 bis 10, **dadurch gekennzeichnet, dass** das Set in einem Kit zusammen mit einem methylierungsspezifischen Restriktionsenzym und/oder einem Reagenz für eine Nukleotid-Desaminierung mit Bisulfit bereitgestellt wird; und/oder wobei das Set Sonden an einem Microarray umfasst.

## Revendications

1. Procédé d'identification d'un cancer colorectal dans un échantillon comprenant de l'ADN d'un sujet, de détermination de l'état de méthylation de deux gènes ou plus comprenant TFPI2 et PTGS2 et de comparaison de l'état de méthylation avec un échantillon de contrôle, en identifiant ainsi de l'ADN du cancer colorectal dans l'échantillon.

2. Procédé selon la revendication 1, **caractérisé en ce que** les un ou plusieurs gènes comprennent les marqueurs de l'un des groupes suivants :
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 et WT1 ;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 et JUB ;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 et S100A2 ;
h) TFPI2, DCC, PTGS2, TJP2, PITX2 ;
i) TFPI2, DCC, PTGS2, TJP2 ;
j) TFPI2, DCC, PTGS2 ;
ou un groupe de marqueurs comprenant au moins 50 %, de préférence au moins 60%, au moins 70 %, au moins 80 %, au moins 90 %, 100 % des gènes de l'un quelconque des groupes a), b), f), h), i), j) ci-dessus, où, pour un groupe donné, au moins les 2 gènes sélectionnés parmi TFPI2 et PTGS2 sont représentés.

3. Procédé selon la revendication 1 ou 2, **caractérisé en ce que** l'étape de détermination de l'état de méthylation comprend une analyse de PCR méthylation-spécifique, une analyse de digestion méthylation-spécifique, de préférence avec une analyse d'hybridation à des fragments non digérés ou digérés, ou une analyse d'amplification par PCR de fragments non digérés ou digérés, ou une désamination par le bisulfite suivie de l'identification de modifications de C méthylées, de préférence par une PCR ou une hybridation.

4. Procédé selon l'une quelconque des revendications 1 à 3, comprenant la comparaison de l'état de méthylation avec le statut d'un état positif et/ou négatif pour le cancer colorectal confirmé.

5. Procédé selon l'une quelconque des revendications 1 à 4, **caractérisé en ce que** l'échantillon du sujet est un échantillon de tissu colorectal, de préférence un polype colorectal, ou un échantillon de sang ou de sérum, particulièrement un échantillon de sang, de plasma ou de sérum prélevé à partir du système circulatoire du sujet.

6. Procédé selon l'une quelconque des revendications 1 à 5, **caractérisé en ce que** l'échantillon de contrôle est un échantillon de tissu colorectal, un échantillon adjacent à un polype colorectal ou un tissu cancéreux, ou un échantillon de sang, de plasma ou de sérum, particulièrement un échantillon comprenant des cellules sanguines, telles que des PBMC.

7. Procédé selon l'une quelconque des revendications 1 à 6, **caractérisé en ce que** l'état de méthylation desdits gènes est déterminé dans une région en amont du cadre de lecture ouvert des gènes marqueurs, en particulier une région promotrice ; ou dans a) un locus génétique défini par les paires d'amorces de SEQ ID NO: 1 et 2, 3 et 4, 5 et 6, 7 et 8, 9 et 10, 11 et 12, 13 et 14, 15 et 16, 17 et 18, 19 et 20, 21 et 22, 23 et 24, 25 et 26, 27 et 28, 29 et 30, 31 et 32, 33 et 34, 35 et 36, 37 et 38, 39 et 40, 41 et 42, 43 et 44, 45 et 46, 47 et 48, 49 et 50, 51 et 52, 53 et 54, 55 et 56, 57 et 58, 59 et 60, 61 et 62, 63 et 64, 65 et 66, 67 et 68, 69 et 70, 71 et 72, 73 et 74, 75 et 76, 77 et 78, 79 et 80, 81 et 82, 83 et 84, 85 et 86, 87 et 88, 89 et 90, 91 et 92, 93 et 94, 95 et 96, et/ou b) le locus chromosomique tel qu'identifié dans le tableau 1 ; ou c) un locus compris à une distance d'une longueur de 1 000 nucléotides au maximum par rapport audit locus a) ou b).

8. Jeu d'amorces d'acide nucléique ou de sondes d'hybridation qui sont spécifiques pour une région potentiellement méthylée de gènes marqueurs qui est appropriée pour diagnostiquer ou prédire le cancer colorectal, le jeu comprenant les sondes ou les amorces pour les gènes :
a) BOLL, CDX1, CLIC4, DCC, ESR1, GATA4, GDNF, HLA-G, JUB, MYOD1, NKX2-1, PENK, PITX2, PTGS2, RARB, S100A2, SALL3, SEZ6L, SFRP2, SPARC, TCEB2, TFPI2, THBD, TJP2, TMEFF2, TP53, TWIST1 et WT1 ;
b) ESR1, TFPI2, WT1, TMEFF2, PENK, MYOD1, TWIST1, DCC, PTGS2, TJP2, SPARC, PITX2, SEZ6L, TP53, GDNF, CDX1, CLIC4, SFRP2, HLA-G, GATA4, BOLL, THBD, RARB, NKX2-1, SALL3 et JUB ;
f) TWIST1, TMEFF2, ESR1, WT1, PITX2, TFPI2, HLA-G, PENK, BOLL, DCC, TP53, GATA4, GDNF, RARB, CLIC4, SEZ6L, SFRP2, PTGS2, THBD, TJP2, SPARC, TCEB2, MYOD1 et S100A2 ;
h) TFPI2, DCC, PTGS2, TJP2, PITX2 ;
i) TFPI2, DCC, PTGS2, TJP2 ;
j) TFPI2, DCC, PTGS2 ;
k) TFPI2, PTGS2 ;
ou des sondes ou des amorces pour au moins 50 %, de préférence au moins 60 %, au moins 70 %, au moins 80 %, au moins 90 %, 100 % des gènes de l'un quelconque de a), b), f), h), i), j) ci-dessus, à condition qu'au moins les 2 gènes sélectionnés parmi TFPI2 et PTGS2 soient représentés,
et l'un quelconque de ces jeux comprend au maximum 350 sondes ou amorces.

9. Jeu selon la revendication 8, **caractérisé en ce que** les paires d'amorces et les sondes sont spécifiques pour une région en amont méthylée du cadre de lecture ouvert des gènes marqueurs, en particulier une région promotrice ; ou sont spécifiques pour a) un locus génétique défini par les paires d'amorces de SEQ ID NO: 1 et 2, 3 et 4, 5 et 6, 7 et 8, 9 et 10, 11 et 12, 13 et 14, 15 et 16, 17 et 18, 19 et 20, 21 et 22, 23 et 24, 25 et 26, 27 et 28, 29 et 30, 31 et 32, 33 et 34, 35 et 36, 37 et 38, 39 et 40, 41 et 42, 43 et 44, 45 et 46, 47 et 48, 49 et 50, 51 et 52, 53 et 54, 55 et 56, 57 et 58, 59 et 60, 61 et 62, 63 et 64, 65 et 66, 67 et 68, 69 et 70, 71 et 72, 73 et 74, 75 et 76, 77 et 78, 79 et 80, 81 et 82, 83 et 84, 85 et 86, 87 et 88, 89 et 90, 91 et 92, 93 et 94, 95 et 96, et/ou b) le locus chromosomique tel qu'identifié dans le tableau 1 ; ou c) un locus compris à une distance d'une longueur de 1 000 nucléotides au maximum par rapport audit locus a) ou b).

10. Jeu selon la revendication 9, **caractérisé en ce que** les sondes ou les amorces sont de SEQ ID NO: 1 à 96.

11. Jeu selon l'une quelconque des revendications 8 à 10, **caractérisé en ce que** le jeu est fourni dans un kit avec une enzyme de restriction méthylation-spécifique et/ou un réactif pour une désamination de nucléotide par le bisulfite ; et/ou dans lequel le jeu comprend des sondes sur un microréseau.


### SEQUENCE LISTING

<110> AIT Austrian Institute of Technology GmbH
<120> Gene methylation based colorectal cancer diagnosis
<130> r66712
<150> EP13198755.4
   <151> 2013-12-20
<160> 96
<170> PatentIn version 3.5
<210> 1
   <211> 21
   <212> DNA
   <213> Artificial
<220>
   <223> primer
<400> 1
   cgcggacgcc gctctgcacc t 21
<210> 2
   <211> 24
   <212> DNA
   <213> Artificial
<220>
   <223> primer
<400> 2
   cggagcccga ctgggctgaa tggc 24
<210> 3
   <211> 21
   <212> DNA
   <213> Artificial
<220>
   <223> primer
<400> 3
   tcggggctca cctggcggga g 21
<210> 4
   <211> 22
   <212> DNA
   <213> Artificial
<220>
   <223> primer
<400> 4
   cccaagtgtc gccgccgctt cc 22
<210> 5
   <211> 20
   <212> DNA
   <213> Artificial
<220>
   <223> primer
<400> 5
   ggcgggcgca ggcaaggtgg 20
<210> 6
   <211> 20
   <212> DNA
   <213> Artificial
<220>
   <223> primer
<400> 6
   gcgccgacca gccgggaagg 20
<210> 7
   <211> 20
   <212> DNA
   <213> Artificial
<220>
   <223> primer
<400> 7
   gtggatgcgg cgcagcgtgg 20
<210> 8
   <211> 19
   <212> DNA
   <213> Artificial
<220>
   <223> primer
<400> 8
   gggccgcgcc cgagcacct 19
<210> 9
   <211> 21
   <212> DNA
   <213> Artificial
<220>
   <223> primer
<400> 9
   tggacgcggg gccacgtctt g 21
<210> 10
   <211> 23
   <212> DNA
   <213> Artificial
<220>
   <223> primer
<400> 10
   acgtgaacgc cgccggattc caa 23
<210> 11
   <211> 21
   <212> DNA
   <213> Artificial
<220>
   <223> primer
<400> 11
   gcctgaaaca cagtaaggtt t 21
<210> 12
   <211> 18
   <212> DNA
   <213> Artificial
<220>
   <223> primer
<400> 12
   cgatctcctg acctcgtg 18
<210> 13
   <211> 25
   <212> DNA
   <213> Artificial
<220>
   <223> primer
<400> 13
   cctgcccagt tactgcccca ctccg 25
<210> 14
   <211> 29
   <212> DNA
   <213> Artificial
<220>
   <223> primer
<400> 14
   tgccgcgaca gttccataac agaaatgaa 29
<210> 15
   <211> 20
   <212> DNA
   <213> Artificial
<220>
   <223> primer
<400> 15
   gtggcgccgg cccgaccagg 20
<210> 16
   <211> 20
   <212> DNA
   <213> Artificial
<220>
   <223> primer
<400> 16
   ccgcccgggg gagcaaggga 20
<210> 17
   <211> 21
   <212> DNA
   <213> Artificial
<220>
   <223> primer
<400> 17
   cgcacaccgc tggcggacac c 21
<210> 18
   <211> 23
   <212> DNA
   <213> Artificial
<220>
   <223> primer
<400> 18
   cgagagccga ggggacccag gga 23
<210> 19
   <211> 21
   <212> DNA
   <213> Artificial
<220>
   <223> primer
<400> 19
   cgccgccgca gctgtcgcct t 21
<210> 20
   <211> 20
   <212> DNA
   <213> Artificial
<220>
   <223> primer
<400> 20
   gccggcctcg cgcaccgtgt 20
<210> 21
   <211> 21
   <212> DNA
   <213> Artificial
<220>
   <223> primer
<400> 21
   ttcagtgttt acacccgcag c 21
<210> 22
   <211> 20
   <212> DNA
   <213> Artificial
<220>
   <223> primer
<400> 22
   ctccaccgga agtgaaaccg 20
<210> 23
   <211> 20
   <212> DNA
   <213> Artificial
<220>
   <223> primer
<400> 23
   gccggggtcg cggactgcca 20
<210> 24
   <211> 21
   <212> DNA
   <213> Artificial
<220>
   <223> primer
<400> 24
   cgcgctgccc cagggattcc a 21
<210> 25
   <211> 20
   <212> DNA
   <213> Artificial
<220>
   <223> primer
<400> 25
   gggcccccgc acccccagaa 20
<210> 26
   <211> 20
   <212> DNA
   <213> Artificial
<220>
   <223> primer
<400> 26
   cccgcgcagc cccagccaag 20
<210> 27
   <211> 21
   <212> DNA
   <213> Artificial
<220>
   <223> primer
<400> 27
   tgggccgcag tgcctcgtgg g 21
<210> 28
   <211> 20
   <212> DNA
   <213> Artificial
<220>
   <223> primer
<400> 28
   gggcgaagcg gccacgggag 20
<210> 29
   <211> 24
   <212> DNA
   <213> Artificial
<220>
   <223> primer
<400> 29
   gggaagagga gacacggaac acca 24
<210> 30
   <211> 24
   <212> DNA
   <213> Artificial
<220>
   <223> primer
<400> 30
   tactcactgg cctcgctctg gttg 24
<210> 31
   <211> 20
   <212> DNA
   <213> Artificial
<220>
   <223> primer
<400> 31
   ctcggccccc tgcaagttcc 20
<210> 32
   <211> 20
   <212> DNA
   <213> Artificial
<220>
   <223> primer
<400> 32
   cgtggcggca ggtgcctgta 20
<210> 33
   <211> 25
   <212> DNA
   <213> Artificial
<220>
   <223> primer
<400> 33
   aaaggcccct tgggaaaccc tcacc 25
<210> 34
   <211> 29
   <212> DNA
   <213> Artificial
<220>
   <223> primer
<400> 34
   ggaaggcaaa ggctgtgaca ggcatcttt 29
<210> 35
   <211> 28
   <212> DNA
   <213> Artificial
<220>
   <223> primer
<400> 35
   ggcattgctc tgcccataga tgcctttg 28
<210> 36
   <211> 26
   <212> DNA
   <213> Artificial
<220>
   <223> primer
<400> 36
   ggaatccctg gttttgacct ggggga 26
<210> 37
   <211> 20
   <212> DNA
   <213> Artificial
<220>
   <223> primer
<400> 37
   gctgagcctg cctgcgcctg 20
<210> 38
   <211> 24
   <212> DNA
   <213> Artificial
<220>
   <223> primer
<400> 38
   tgagcgaccc ctccagtagc ccaa 24
<210> 39
   <211> 20
   <212> DNA
   <213> Artificial
<220>
   <223> primer
<400> 39
   cgcccagcgg gcaccaccag 20
<210> 40
   <211> 20
   <212> DNA
   <213> Artificial
<220>
   <223> primer
<400> 40
   gtggcggcct tgcggcggtc 20
<210> 41
   <211> 20
   <212> DNA
   <213> Artificial
<220>
   <223> primer
<400> 41
   gctgccgggc cgccctccct 20
<210> 42
   <211> 19
   <212> DNA
   <213> Artificial
<220>
   <223> primer
<400> 42
   actgcggggc cgcgggctg 19
<210> 43
   <211> 22
   <212> DNA
   <213> Artificial
<220>
   <223> primer
<400> 43
   ctttctgggg ccccgccctc cc 22
<210> 44
   <211> 20
   <212> DNA
   <213> Artificial
<220>
   <223> primer
<400> 44
   cctcgcgttg ggggcgaccg 20
<210> 45
   <211> 25
   <212> DNA
   <213> Artificial
<220>
   <223> primer
<400> 45
   acgtggctcc gcaccaatca ggacg 25
<210> 46
   <211> 20
   <212> DNA
   <213> Artificial
<220>
   <223> primer
<400> 46
   cccagtgcgc acggcgaggc 20
<210> 47
   <211> 20
   <212> DNA
   <213> Artificial
<220>
   <223> primer
<400> 47
   gcgggtgcgg ggatcggggt 20
<210> 48
   <211> 22
   <212> DNA
   <213> Artificial
<220>
   <223> primer
<400> 48
   caggccagag gcaggcccgc ag 22
<210> 49
   <211> 20
   <212> DNA
   <213> Artificial
<220>
   <223> primer
<400> 49
   cgccaggacc gcgcacagca 20
<210> 50
   <211> 25
   <212> DNA
   <213> Artificial
<220>
   <223> primer
<400> 50
   cacagccaga cgccctcaga cagca 25
<210> 51
   <211> 20
   <212> DNA
   <213> Artificial
<220>
   <223> primer
<400> 51
   cgggcgcagg cggaacaccg 20
<210> 52
   <211> 25
   <212> DNA
   <213> Artificial
<220>
   <223> primer
<400> 52
   cgggacgctg ctcctggctc acgtt 25
<210> 53
   <211> 19
   <212> DNA
   <213> Artificial
<220>
   <223> primer
<400> 53
   ggcgggggct gcggctgct 19
<210> 54
   <211> 24
   <212> DNA
   <213> Artificial
<220>
   <223> primer
<400> 54
   ccctgagggc ggtgtgaacc acga 24
<210> 55
   <211> 20
   <212> DNA
   <213> Artificial
<220>
   <223> primer
<400> 55
   cggccccgcg ggattttgcc 20
<210> 56
   <211> 27
   <212> DNA
   <213> Artificial
<220>
   <223> primer
<400> 56
   ccagagggtc tggcggacaa gaaaggg 27
<210> 57
   <211> 26
   <212> DNA
   <213> Artificial
<220>
   <223> primer
<400> 57
   gggtggggag gtaggagaag cggaaa 26
<210> 58
   <211> 25
   <212> DNA
   <213> Artificial
<220>
   <223> primer
<400> 58
   tgccagcagc ccagaaaaag agccc 25
<210> 59
   <211> 20
   <212> DNA
   <213> Artificial
<220>
   <223> primer
<400> 59
   cccgccgccc ggccagcttt 20
<210> 60
   <211> 22
   <212> DNA
   <213> Artificial
<220>
   <223> primer
<400> 60
   caggctgcgt caacgcgctg gg 22
<210> 61
   <211> 28
   <212> DNA
   <213> Artificial
<220>
   <223> primer
<400> 61
   tgttgagctg tcaatgtgtg gttaaaaa 28
<210> 62
   <211> 18
   <212> DNA
   <213> Artificial
<220>
   <223> primer
<400> 62
   tcaggcaatc cgcccacc 18
<210> 63
   <211> 20
   <212> DNA
   <213> Artificial
<220>
   <223> primer
<400> 63
   ctccgcgccc cctgcagcca 20
<210> 64
   <211> 21
   <212> DNA
   <213> Artificial
<220>
   <223> primer
<400> 64
   cgccgggctc cccaggagca g 21
<210> 65
   <211> 20
   <212> DNA
   <213> Artificial
<220>
   <223> primer
<400> 65
   cccgggcccg ctctcttcgc 20
<210> 66
   <211> 20
   <212> DNA
   <213> Artificial
<220>
   <223> primer
<400> 66
   cgcctcgccc gcgctgtcct 20
<210> 67
   <211> 22
   <212> DNA
   <213> Artificial
<220>
   <223> primer
<400> 67
   gtcccccatc cgcccccaac tg 22
<210> 68
   <211> 25
   <212> DNA
   <213> Artificial
<220>
   <223> primer
<400> 68
   cccactgcgg ttaccccgca tgctc 25
<210> 69
   <211> 21
   <212> DNA
   <213> Artificial
<220>
   <223> primer
<400> 69
   aggctgggcg ggggtcacac a 21
<210> 70
   <211> 22
   <212> DNA
   <213> Artificial
<220>
   <223> primer
<400> 70
   cccctccaca ttcccgcggt cc 22
<210> 71
   <211> 25
   <212> DNA
   <213> Artificial
<220>
   <223> primer
<400> 71
   cgtgtgttct gggcagggtt tgagg 25
<210> 72
   <211> 21
   <212> DNA
   <213> Artificial
<220>
   <223> primer
<400> 72
   gctctcccag ctgcacgcca a 21
<210> 73
   <211> 18
   <212> DNA
   <213> Artificial
<220>
   <223> primer
<400> 73
   ggcccgcggc tctgtgcg 18
<210> 74
   <211> 21
   <212> DNA
   <213> Artificial
<220>
   <223> primer
<400> 74
   gtgtcggatc cgcaggcgca g 21
<210> 75
   <211> 20
   <212> DNA
   <213> Artificial
<220>
   <223> primer
<400> 75
   cgtgcggccg ccatcccgac 20
<210> 76
   <211> 20
   <212> DNA
   <213> Artificial
<220>
   <223> primer
<400> 76
   gcccgccgga ccgcaccaac 20
<210> 77
   <211> 21
   <212> DNA
   <213> Artificial
<220>
   <223> primer
<400> 77
   gacagcccca gggggcgagc g 21
<210> 78
   <211> 20
   <212> DNA
   <213> Artificial
<220>
   <223> primer
<400> 78
   cgcgcacctc ctcccgccag 20
<210> 79
   <211> 21
   <212> DNA
   <213> Artificial
<220>
   <223> primer
<400> 79
   cgggtgggga agtcgcgggg a 21
<210> 80
   <211> 21
   <212> DNA
   <213> Artificial
<220>
   <223> primer
<400> 80
   ccctacccgg cgccgcagca a 21
<210> 81
   <211> 20
   <212> DNA
   <213> Artificial
<220>
   <223> primer
<400> 81
   tgtgccgcgc ggttgggagg 20
<210> 82
   <211> 24
   <212> DNA
   <213> Artificial
<220>
   <223> primer
<400> 82
   cagcttccta cggcgcatcg ggga 24
<210> 83
   <211> 24
   <212> DNA
   <213> Artificial
<220>
   <223> primer
<400> 83
   tccaggggca gacgagtgga gccc 24
<210> 84
   <211> 22
   <212> DNA
   <213> Artificial
<220>
   <223> primer
<400> 84
   ccagagggat gcgggcggca ga 22
<210> 85
   <211> 20
   <212> DNA
   <213> Artificial
<220>
   <223> primer
<400> 85
   ccgcgggttc cgtgggtcgc 20
<210> 86
   <211> 22
   <212> DNA
   <213> Artificial
<220>
   <223> primer
<400> 86
   caccgcgggt cgctacgggc ct 22
<210> 87
   <211> 22
   <212> DNA
   <213> Artificial
<220>
   <223> primer
<400> 87
   ccaacggctg gacgcacacc cc 22
<210> 88
   <211> 28
   <212> DNA
   <213> Artificial
<220>
   <223> primer
<400> 88
   ccaagtctgc agctctcgcc caactccc 28
<210> 89
   <211> 22
   <212> DNA
   <213> Artificial
<220>
   <223> primer
<400> 89
   tgtcccagct gagccccgcg tt 22
<210> 90
   <211> 24
   <212> DNA
   <213> Artificial
<220>
   <223> primer
<400> 90
   gcgggaccag gttctaccgc aggc 24
<210> 91
   <211> 20
   <212> DNA
   <213> Artificial
<220>
   <223> primer
<400> 91
   aggcggcaaa acccgccatc 20
<210> 92
   <211> 21
   <212> DNA
   <213> Artificial
<220>
   <223> primer
<400> 92
   tgccgcaggg acaatatggc a 21
<210> 93
   <211> 25
   <212> DNA
   <213> Artificial
<220>
   <223> primer
<400> 93
   cgaccgccac aaggaggaca acgga 25
<210> 94
   <211> 22
   <212> DNA
   <213> Artificial
<220>
   <223> primer
<400> 94
   tgcgttgggc gacggcgacc tt 22
<210> 95
   <211> 23
   <212> DNA
   <213> Artificial
<220>
   <223> primer
<400> 95
   agctcgctgc tgacgcacta ccg 23
<210> 96
   <211> 18
   <212> DNA
   <213> Artificial
<220>
   <223> primer
<400> 96
   ggcgcaggcg ctcccaga 18